(19) Europäisches Patentamt European Patent Office Office européen des brevets

(11) **EP 3 872 483 B1**

(12) **EUROPEAN PATENT SPECIFICATION**

(45) Date of publication and mention of the grant of the patent:
**18.10.2023 Bulletin 2023/42**

(21) Application number: **19877043.0**

(22) Date of filing: **18.10.2019**

(51) International Patent Classification (IPC):
**G01N 27/02** *(2006.01)*    **G01N 33/24** *(2006.01)*

(52) Cooperative Patent Classification (CPC):
**G01N 33/246; G01N 27/02**

(86) International application number:
**PCT/JP2019/041187**

(87) International publication number:
**WO 2020/085250 (30.04.2020 Gazette 2020/18)**

(54) **SOIL EVALUATION SENSOR, SOIL EVALUATION SYSTEM, ELECTRODE FOR SOIL EVALUATION SENSOR, AND DEVICE FOR OBTAINING IMPEDANCE CHARACTERISTIC OF SOIL**

BODENAUSWERTUNGSSENSOR, BODENAUSWERTUNGSSYSTEM, ELEKTRODE FÜR EINEN BODENAUSWERTUNGSSENSOR UND VORRICHTUNG ZUR GEWINNUNG VON IMPEDANZCHARAKTERISTIKA DES BODENS

CAPTEUR D'ÉVALUATION DES SOLS, SYSTÈME D'ÉVALUATION DES SOLS, ÉLECTRODE POUR CAPTEUR D'ÉVALUATION DES SOLS, ET DISPOSITIF POUR OBTENIR UNE CARACTÉRISTIQUE D'IMPÉDANCE DES SOLS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2018 JP 2018198529**

(43) Date of publication of application:
**01.09.2021 Bulletin 2021/35**

(73) Proprietor: **National University Corporation Shizuoka University Shizuoka-shi, Shizuoka 422-8529 (JP)**

(72) Inventor: **FUTAGAWA, Masato Hamamatsu-shi, Shizuoka 432-8561 (JP)**

(74) Representative: **Hoffmann Eitle Patent- und Rechtsanwälte PartmbB Arabellastraße 30 81925 München (DE)**

(56) References cited:
EP-A1- 2 584 348      WO-A1-2018/110219
JP-A- 2003 090 869    JP-A- 2004 198 385
JP-A- 2004 198 385    JP-A- 2013 040 878
JP-A- 2013 200 193    JP-A- 2013 200 193
JP-A- 2015 197 363    JP-A- 2018 128 414
US-A- 4 654 598

• Ogasawara ,Shin: "24pm3-B-6: Fabrication of a Soil Impedance Sensor with a Shielding Electrode to Measure of Low-Level Soil Water Content", 33rd Sensor Symposium on Sensors, Micromachines and Applied systems; Hirado (Japan); October 24-26, 2016, vol. 33, 17 October 2016 (2016-10-17), pages 1-5, XP009527681,
• Nagaya Shogo: "02-pml-PS-220: Fabrication and Evaluation of an Impedance Measurement Circuit Chip to Measure Water Content and Ion Concentration in Culture Medium Soil", 34th Sensor Symposium on Sensors, Micromachines and Applied systems; Hiroshima; October 31st - November 2nd, 2017, vol. 34, 24 October 2017 (2017-10-24), pages 1-5, XP009527682,

## Description

## Technical Field

The present invention relates to a soil evaluation sensor, a soil evaluation system, an electrode for a soil evaluation sensor, and a device for obtaining the impedance characteristic of soil.

## Background Art

Patent Literature 1 discloses a device for obtaining an evaluation value of soil, or the like. The device disclosed in Patent Literature 1 accurately and inexpensively detects the water content and the electrical conductivity of soil exemplified as evaluation values. The device disclosed in Patent Literature 1 applies an AC input electrical signal to one of the electrodes disposed on the soil. The other electrode disposed on the soil captures an output electrical signal due to the input electrical signal. The output electrical signal includes information regarding the impedance of the soil present between the electrodes. Therefore, the device disclosed in Patent Literature 1 includes an absolute value detection circuit for detecting the absolute value of impedance and a phase detection circuit for detecting the phase of impedance. The device disclosed in Patent Literature 1 obtains the absolute value and the phase as information regarding impedance by using the outputs of these detection circuits. Then, the device disclosed in Patent Literature 1 obtains the water content and the electrical conductivity of the soil by using the absolute value and the phase.

## Citation List

## Patent Literature

Patent Literature 1: Japanese Unexamined Patent Publication No. 2013-200193

## Summary of Invention

## Technical Problem

The device disclosed in Patent Literature 1 obtains both the absolute value and the phase of the impedance of the soil present between the electrodes by measurement. That is, the water content and the like of the soil are calculated using the absolute value and the phase of the impedance, which are measured values. According to this configuration, the accuracy of the evaluation value such as the water content of the soil is affected by the measurement accuracy of the absolute value and the phase of the impedance.

Therefore, the present invention provides a soil evaluation system in accordance with the independent claim for obtaining the impedance characteristic of soil, which are not easily affected by the measurement accuracy included in a measured value for obtaining the evaluation value of the soil.

## Solution to Problem

The present invention relates to a soil evaluation system that evaluates the characteristics of soil. The soil evaluation system includes: a first sensor unit having a first electrode portion that includes a pair of electrode ends disposed in the soil and that provides an AC input electrical signal to the soil and receives an output electrical signal from the soil; a second sensor unit that has a second electrode portion, which includes a pair of electrode ends disposed in the soil and which provides an AC input electrical signal to the soil and receives an output electrical signal from the soil, and that is spaced apart from the first sensor unit; and a control unit that provides a control signal for controlling operations of the first sensor unit and the second sensor unit to the first sensor unit and the second sensor unit, acquires first information indicating a relationship between a frequency of the input electrical signal and an absolute value of impedance of the soil by using the input electrical signal and the output electrical signal, calculates second information indicating a relationship between the frequency of the input electrical signal and a phase of the impedance of the soil by using the first information, and obtains an evaluation value of the soil by using the first information and the second information. The control unit performs a first operation to provide the input electrical signal to the soil from one of the electrode ends included in the first electrode portion and acquire the output electrical signal due to the input electrical signal from the other electrode end included in the first electrode portion and a second operation to provide the input electrical signal to the soil from the first electrode portion and acquire the output electrical signal due to the input electrical signal from the second electrode portion.

The soil evaluation system performs a first operation of operating the first sensor unit and the second sensor unit independently and a second operation of operating the first sensor unit and the second sensor unit in cooperation with each other. As a result, it is possible to generate a measurement range by the first operation and a measurement range by the second operation. Therefore, it is possible to form a measurement range without leakage for a wide target region. In addition, the control unit obtains the phase of impedance by calculation from the absolute value of the impedance. According to this process, it is not necessary to timely synchronize the timing of providing the input electrical signal from the first sensor unit and the timing of obtaining the output electrical signal by the second sensor unit at the time of the second operation to operate the first sensor unit and the second sensor unit in cooperation with each other. Therefore, a measurement error due to phase measurement does not occur. As a result, the soil evaluation system can obtain a good evaluation value by making the first sensor unit and the second sensor unit cooperate with each other.

The present invention relates to a soil evaluation system for evaluating the characteristics of soil in a target region. The soil evaluation system includes: a first sensor unit that includes a pair of electrode ends disposed in the soil and is able to provide an AC input electrical signal to the soil; a second sensor unit that includes a pair of electrode ends disposed in the soil, is able to acquire an output electrical signal due to the input electrical signal from the soil, and is spaced apart from the first sensor unit; and a control unit that provides a control signal for controlling operations of the first sensor unit and the second sensor unit to the first sensor unit and the second sensor unit, acquires first information indicating a relationship between a frequency of the input electrical signal and an absolute value of impedance of the soil by using the input electrical signal and the output electrical signal, calculates second information indicating a relationship between the frequency of the input electrical signal and a phase of the impedance of the soil by using the first information, and obtains an evaluation value of the soil by using the first information and the second information.

Also with this configuration, the soil evaluation system can suppress the influence of the measurement accuracy included in the measured value for obtaining the evaluation value of the soil. In addition, the soil evaluation system can obtain a good evaluation value.

In still another aspect, the soil evaluation system may further include a third sensor unit that includes a pair of electrode ends disposed in the soil, is able to provide the input electrical signal and acquire the output electrical signal, and is spaced apart from the first sensor unit and the second sensor unit. The first sensor unit may be able to further acquire the output electrical signal. The second sensor unit may be able to further provide the input electrical signal. The first sensor unit, the second sensor unit, and the third sensor unit may be disposed so as to surround a target region defined as a two-dimensional surface. According to this configuration, it is possible to obtain a good evaluation value for the two-dimensional target region.

In still another aspect, the control unit may perform a first operation of providing the input electrical signal from the first sensor and acquiring the output electrical signal from the second sensor. After the first operation, the control unit may perform a second operation of providing the input electrical signal from the first sensor and acquiring the output electrical signal from the third sensor unit. After the second operation, the control unit may perform a third operation of providing the input electrical signal from the second sensor and acquiring the output electrical signal from the third sensor unit. According to this configuration, it is possible to obtain the evaluation value by so-called time division processing.

In still another embodiment, the target region may include a plurality of partial regions, and a first weighting coefficient, a second weighting coefficient, and a third weighting coefficient may be set for the plurality of partial regions. The control unit may perform an operation of obtaining a first calculation value by using the first weighting coefficient and the evaluation value obtained as a result of the first operation, an operation of obtaining a second calculation value by using the second weighting coefficient and the evaluation value obtained as a result of the second operation, an operation of obtaining a third calculation value by using the third weighting coefficient and the evaluation value obtained as a result of the third operation, and an operation of obtaining a total calculation value for each of the partial regions by using the first calculation value, the second calculation value, and the third calculation value obtained for each of the partial regions. According to this configuration, it is possible to present the evaluation value in a visually easy-to-understand manner.

In still another aspect, the soil evaluation system may further include: a third sensor unit that includes a pair of electrode ends disposed in the soil, is able to provide the input electrical signal and acquire the output electrical signal, and is spaced apart from the first sensor unit and the second sensor unit; and a fourth sensor unit that includes a pair of electrode ends disposed in the soil, is able to provide the input electrical signal and acquire the output electrical signal, and is spaced apart from the first sensor unit, the second sensor unit, and third sensor unit. The first sensor unit may be able to further acquire the output electrical signal. The second sensor unit may be able to further provide the input electrical signal. The first sensor unit, the second sensor unit, the third sensor unit, and the fourth sensor unit may be disposed so as to surround a target region defined as a three-dimensional region. According to this configuration, it is possible to obtain a good evaluation value for the three-dimensional target region.

## Advantageous Effects of Invention

According to the present invention, there is provided a soil evaluation system, for obtaining the impedance characteristic, which are not easily affected by the measurement accuracy included in the measured value for obtaining the evaluation value of soil.

## Brief Description of Drawings

FIG. 1 is a diagram showing a functional block example of a soil evaluation sensor.
FIG. 2(a) is a graph showing a relationship between the frequency of an AC voltage and the absolute value of impedance.
FIG. 2(b) is a graph showing a relationship between the frequency of an AC voltage and the phase of impedance.

FIG. 3 is a functional block diagram of a soil evaluation system using the soil evaluation sensor in FIG. 1.

FIG. 4(a) is a diagram showing a first measurement range of the soil evaluation system.

FIG. 4(b) is a diagram showing a second measurement range of the soil evaluation system.

FIG. 4(c) is a diagram showing a third measurement range of the soil evaluation system.

FIG. 4(d) is a diagram showing a fourth measurement range of the soil evaluation system.

FIG. 4(e) is a diagram showing the measurement range of the soil evaluation system.

FIG. 5(a) is a diagram showing a functional block of a soil evaluation sensor of a first modification example.

FIG. 5(b) is a diagram showing a first connection configuration of the soil evaluation sensor.

FIG. 6(a) is a diagram showing a second connection configuration of the soil evaluation sensor.

FIG. 6(b) is a diagram showing a third connection configuration of the soil evaluation sensor.

FIG. 7 is a functional block diagram showing a first mode in a soil evaluation system using the soil evaluation sensor of the first modification example.

FIG. 8 is a functional block diagram showing a second mode in the soil evaluation system using the soil evaluation sensor of the modification example.

FIG. 9 is a functional block diagram of a soil evaluation system of a second modification example.

FIG. 10 is a perspective view showing the configuration of a soil evaluation system of a third modification example.

FIG. 11 is a diagram showing a main part of the soil evaluation system of the third modification example.

FIG. 12 is a perspective view showing the configuration of a soil evaluation system of a fourth modification example.

FIGS. 13(a) and 13(b) are perspective views showing a soil evaluation system of a sixth modification example.

FIG. 14 is a perspective view showing a soil evaluation system of a seventh modification example.

FIG. 15 is a map presented by a soil evaluation system of an eighth modification example.

FIGS. 16(a), 16(b), 16(c), and 16(d) are diagrams for explaining a process of obtaining a map.

FIGS. 17(a) and 17(b) are diagrams for explaining a process of obtaining a map.

FIGS. 18(a) and 18(b) are diagrams for explaining a process of obtaining a map.

FIGS. 19(a) and 19(b) are diagrams for explaining a process of obtaining a map.

## Description of Embodiments

Hereinafter, embodiments for carrying out the present invention will be described in detail with reference to the accompanying diagrams. In the description of the diagrams, the same elements are denoted by the same reference numerals, and the repeated description thereof will be omitted.

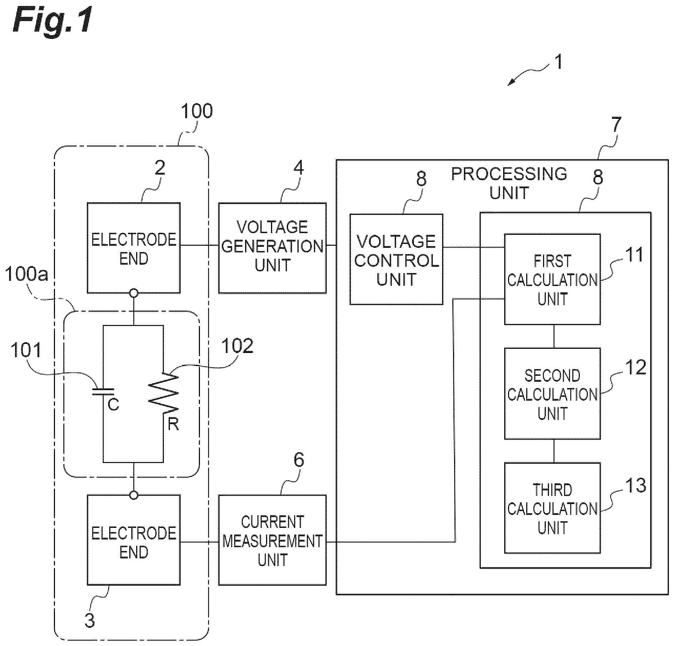

FIG. 1 is a functional block diagram showing an example of a configuration of a soil evaluation sensor 1. The soil evaluation sensor 1 is disposed on soil 100. The soil evaluation sensor 1 obtains an evaluation value for evaluating the characteristics of a partial region of the soil 100a to be evaluated. In the following description, a partial region of the soil 100a to be evaluated is simply referred to as "soil 100a" (measurement target). Examples of the evaluation value include the amount of water contained in the soil 100a and the total ion concentration. However, the evaluation value is not limited to the water content and the total ion concentration.

The soil evaluation sensor 1 obtains an impedance characteristic with respect to the soil 100a to be measured. The impedance characteristic includes the absolute value of impedance and the phase of impedance. The soil 100a can be modeled as an electrical circuit having a capacitance 101 and an electrical resistance 102 configuring an impedance. The capacitance 101 and the electrical resistance 102 are connected in parallel with each other. For example, the value of the capacitance of the soil 100a is 10 pF (picofarad) or more and 500 pF or less. The value of the electrical resistance of the soil 100a is 100 S2 (ohm) or more and 1 MS2 (megaohm) or less. Therefore, the soil evaluation sensor 1 measures the value of the capacitance and the value of the electrical resistance in the wide ranges exemplified above. The value of the capacitance and the value of the electrical resistance are related to the water content and the total ion concentration of the soil 100a. As a result, it is possible to obtain the water content and the total ion concentration by obtaining the value of the capacitance and the value of the electrical resistance. The water content refers to the ratio of the volume occupied by water in the soil 100a per unit volume. The total ion concentration refers to the sum of some ions contained in water. For example, in the agricultural field, the total ion concentration indicates the sum of main ions such as nitrogen ion, phosphate ion, and potassium ion.

The soil evaluation sensor 1 includes an electrode end 2 (first electrode), an electrode end 3 (second electrode), a voltage generation unit 4 (signal generation unit, first signal generation unit, second signal generation unit), a current measurement unit 6 (signal measurement unit, first signal measurement unit, second signal measurement unit), and a processing unit 7. The soil evaluation sensor 1 may be an electrical circuit that realizes these functions. The soil evaluation sensor 1 may be a sensor chip integrated on a semiconductor substrate.

In addition, in the form illustrated below, an AC voltage is exemplified as an input electrical signal (input electrical signal). An AC current is exemplified as an output electrical signal (output electrical signal). However, the input electrical signal and the output electrical signal are not limited to the AC voltage and the AC current. As the input electrical signal, either voltage or current may be adopted. As the output electrical signal, either voltage or current may be adopted. A combination thereof may be an appropriate combination according to the measurement target. For example, a current may be selected as the input electrical signal.

The signal generation unit and the signal measurement unit correspond to the modes of the input electrical signal and the output electrical signal. When a voltage is adopted as an input electrical signal, the signal generation unit is a voltage generation unit (voltage source). When a current is adopted as an input electrical signal, the signal generation unit is a current generation unit (current source). When a voltage is adopted as an output electrical signal, the signal measurement unit is a voltage measurement unit (voltmeter). When a current is used as the output electrical signal, the signal measurement unit is a current measurement unit (ammeter).

The electrode end 2 (first electrode) provides an AC voltage (input electrical signal) to the soil 100a. The electrode end 2 is a metal plate-shaped member. The electrode end 2 is, for example, an electrode pad provided on a sensor chip. The electrode end 3 (second electrode) receives an AC current (output electrical signal) from the soil 100a. The AC current is due to the AC voltage provided from the electrode end 2. The electrode end 3 is a metal plate-shaped member, similarly to the electrode end 2. The electrode end 3 is provided so as to be spaced apart from the electrode end 2. The soil 101a to be measured is interposed between the electrode ends 2 and 3.

The voltage generation unit 4, which is a voltage source, is electrically connected to the electrode end 2. The voltage generation unit 4 provides an AC voltage to the electrode end 2. The AC voltage is defined by a predetermined amplitude (AC voltage value) and a predetermined frequency. The voltage generation unit 4 can change the amplitude and the frequency within a predetermined range. The mode of the AC voltage output from the voltage generation unit 4 is controlled by a voltage control unit 8 of the processing unit 7, which will be described later.

The current measurement unit 6, which is an ammeter, is electrically connected to the electrode end 3. The current measurement unit 6 measures the AC current received by the electrode end 3. The current measurement unit 6 obtains the amplitude (current value) of the AC current. The current measurement unit 6 is connected to a first calculation unit 11 of the processing unit 7, which will be described later. The current measurement unit 6 provides the first calculation unit 11 with information (current value) regarding the AC current.

The processing unit 7 controls the operation of the voltage generation unit 4. In addition, the processing unit 7 calculates an evaluation value of the soil 100a. The processing unit 7 is connected to the voltage generation unit 4 and the current measurement unit 6. The processing unit 7 provides the voltage generation unit 4 with a control signal for controlling the frequency of the AC voltage. The processing unit 7 obtains an evaluation value indicating the characteristics of the soil by using the AC current obtained by the current measurement unit 6.

The processing unit 7 may be a computer having a calculation function. The processing unit 7 is, for example, a personal computer, a specially designed microcomputer, or a mobile information terminal such as a smartphone. In these computer systems, a program for realizing each functional block included in the processing unit 7 is executed. As a result, the processing unit 7 is realized.

The processing unit 7 includes the voltage control unit 8 and an evaluation value calculation unit 9 as functional components.

The voltage control unit 8 generates a control signal for controlling the operation of the voltage generation unit 4. The voltage control unit 8 provides the control signal to the voltage generation unit 4. For example, the voltage control unit 8 causes the voltage generation unit 4 to output a value selected from 10 mV or more and 500 mV or less as the amplitude of the AC voltage. The voltage control unit 8 causes the voltage generation unit 4 to output a value of $10^2$ Hz or more and $10^8$ Hz or less as the frequency of the AC voltage. For the predetermined frequency, at least two different values are selected in this frequency band. In addition, the predetermined frequency may be a plurality of values discretely selected from the predetermined frequency band. The predetermined frequency may be obtained by continuously changing the frequency within the predetermined frequency band. It is desirable to perform measurement at a point where a large change in the AC current, which is an output electrical signal, with respect to a change in the frequency of the AC voltage is likely to occur. For example, when setting two values as the frequency of the AC voltage, a first value is adopted from the band (S1) in FIG. .2(a). In addition, a second value may be adopted from the band (S3). A point where the difference between the output electrical signals is large may be found to perform measurement. For example, the first value and the second value are set such that the difference between the output electrical signal (AC current) obtained at the frequency of the first value and the output electrical signal (AC current) obtained at the frequency of the second value is larger than a predetermined value. Then, the measurement may be performed based on the setting.

The evaluation value calculation unit 9 may include the first calculation unit 11, a second calculation unit 12, and a third calculation unit 13.

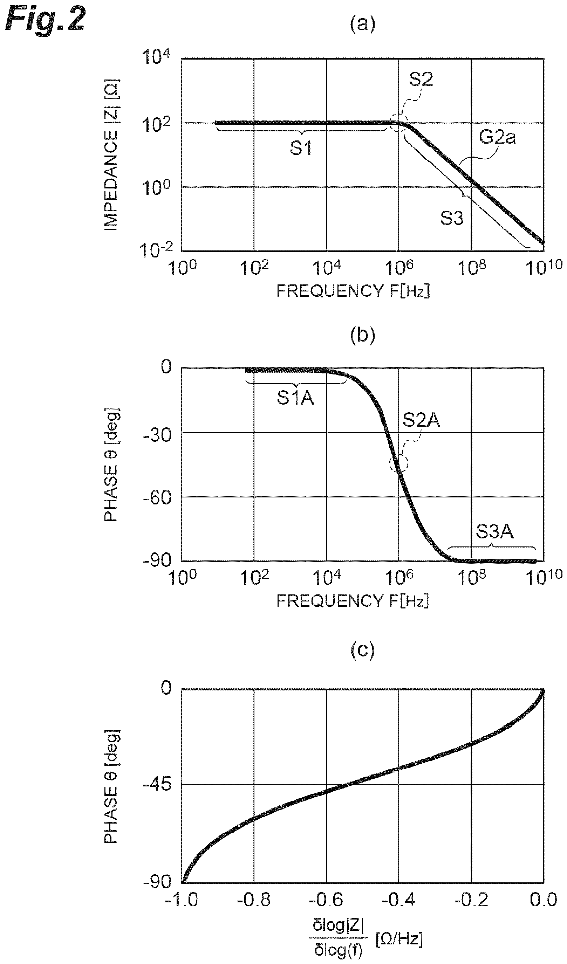

The first calculation unit 11 obtains first information (see FIG. 2(a)) by using the AC voltage and the AC current. The first information indicates a relationship between the frequency of the AC voltage and the absolute value of the impedance of the soil 100a. The first information may be a collection of a plurality of pieces of data in which a frequency and an absolute value are associated with each other. For example, the first information may be a data set represented as at least [frequency ($\omega$1), absolute value |Z1|] and [(frequency ($\omega$2), absolute value |Z2|]. The first information includes a relationship between two different frequencies and the absolute value of the impedance at each frequency.

The first information may be a function in which the frequency is an independent variable and the absolute value is a dependent variable. The first calculation unit 11 obtains the frequency of the AC voltage from the voltage control unit 8. The first calculation unit 11 may obtain the frequency of the AC voltage from the voltage generation unit 4. The first calculation unit 11 may obtain the frequency of the AC voltage from a voltmeter (not shown) connected to the electrode end 2. The first calculation unit 11 calculates the absolute value of the impedance by using the amplitude of the AC current provided by the current measurement unit 6. The absolute value of impedance may be calculated by a functional component other than the first calculation unit 11.

The second calculation unit 12 calculates second information (see FIG. 2(b)) using the first information. The second information indicates a relationship between the frequency of the AC voltage and the phase of the impedance of the soil 100a. In general, when measuring impedance, the absolute value and the phase are obtained by measurement. The soil evaluation sensor 1 obtains the absolute value of impedance by measurement. However, the soil evaluation sensor 1 obtains the phase of impedance by calculation. The soil evaluation sensor 1 is not directly measure the phase of impedance. The second calculation unit 12 calculates the second information by using the relationship between the frequency of the AC voltage and the absolute value of the impedance shown as the first information.

The soil 100a can be modeled as a parallel circuit (see FIG. 1) of the electrical resistance 102 and the capacitance 101. The circuit model does not depend on the properties of the soil 100a. In other words, even if the properties of the soil 100a change, the soil 100a may be modeled as a parallel circuit of the electrical resistance 102 and the capacitance 101.

Referring to a graph G2a shown in FIG. 2(a), the phase is 0° in the band (S1) in which the absolute value (|Z|) does not change with respect to the increase in frequency (S1A). At the inflection point (S2) of the absolute value (|Z|), the phase is -45° (S2A). In the band (S3) in which the absolute value (|Z|) changes with a slope of 1/f, the phase is -90° (S3A). That is, the phase of impedance changes substantially monotonously with respect to the absolute value of impedance. In other words, the phase of impedance decreases uniformly with respect to the change in the absolute value of impedance. The relationship between the absolute value and the phase is the same even if the value of electrical resistance and the value of capacitance change. As a result, since the relationship between the absolute value and the phase does not depend on the type and water content of the soil 100a, it is possible to estimate the phase only from the absolute value of the impedance. For example, the absolute value of the impedance with respect to the first frequency and the absolute value of the impedance with respect to the second frequency different from the first frequency may be used for the phase estimation. Equation (1) shows the absolute value of impedance. Equation (2) indicates the phase of impedance. In equation (1), |Z| is the absolute value of impedance. R is an electrical resistance. C is a capacitance. $\omega$ is an angular frequency. $\theta$ is the phase of impedance.

[Equation 1]

$$|Z| = \frac{\sqrt{R^2 + \omega^2 R^4 C^2}}{\omega^2 R^2 C^2 + 1} \quad \cdots \quad (1)$$

[Equation 2]

$$\theta = \tan^{-1}(\omega C R) \quad \cdots \quad (2)$$

A result obtained by differentiating Equation (1) by the angular frequency ($\omega$) is defined as D. By substituting Equation (1) into Equation (3), Equation (4) is obtained.

[Equation 3]

$$D = \frac{\partial \log|Z|}{\partial \log \omega} \cdots \quad (3)$$

[Equation 4]

$$D = -\frac{R^2 C^2 \omega^2}{R^2 C^2 \omega^2 + 1} \quad \cdots \quad (4)$$

By using Equation (2) and Equation (4), the electrical resistance (R), the capacitance (C), and the angular frequency ($\omega$) are eliminated. As a result, Equation (5) is obtained.

[Equation 5]

$$\theta = -\tan^{-1} \sqrt{-\frac{D}{D+1}} \quad \cdots \quad (5)$$

For example, it is assumed that the first information is a data set represented as [frequency ($\omega$1), absolute value |Z1|), (frequency ($\omega$2), absolute value |Z2|)]. In this case, the second calculation unit 12 may calculate the real part and the imaginary part of the impedance by difference calculation. In addition, it is assumed that the "differential calculation" in the soil evaluation sensor 1 includes a difference calculation.

From Equation (5), it can be seen that the phase

($\theta$) of impedance is uniquely determined with respect to the frequency derivative of the absolute value of impedance. The function (D) is a function of the absolute value of impedance. Therefore, the function (D) can be measured without the need for time synchronization. As a result, the phase can be estimated by using the above equation.

FIG. 2(c) is a graph of Equation (5). The horizontal axis indicates D in Equation (5). The vertical axis indicates the phase ($\theta$) in Equation (5). According to the graph shown in FIG. 2(c), it can be visually confirmed that the phase ($\theta$) is a function of D. In addition, D is a function of the absolute value |Z| of impedance as described above. Therefore, the phase ($\theta$) of impedance can be obtained by obtaining the absolute value |Z| of the impedance. For example, D on the horizontal axis is converted to the angular frequency ($\omega$) using Equation (4), and then the angular frequency ($\omega$) is further changed to the frequency (f), so that a graph corresponding to the graph shown in FIG. 2(b) is obtained.

The third calculation unit 13 obtains the evaluation value of the soil 100a by using the first information and the second information. The first information and the second information may be the relationship between the frequency of the AC voltage and the absolute value and phase of the impedance. The first information and the second information may be the electrical resistance and the capacitance derived from the relationship between the frequency of the AC voltage and the absolute value and phase of the impedance. For example, the electrical resistance can be obtained from the band (S1) in which the absolute value (|Z|) does not change in the first information. The capacitance can be obtained from the phase of impedance. As described above, the water content and the total ion concentration of the soil 100a are related to the value of the capacitance and the value of the electrical resistance. The third calculation unit 13 divides the value of the capacitance of the soil 100a by the relative permittivity. As a result, the third calculation unit 13 obtains the water content. The third calculation unit 13 divides the value of the electrical resistance by the water content. As a result, the third calculation unit 13 obtains the total ion concentration.

The soil evaluation sensor 1 obtains an AC current due to the AC voltage, which is provided from the electrode end 2, from the electrode end 3. The AC voltage and the AC current reflect the characteristics of the soil 100a that can be modeled as a parallel circuit of electrical resistance and capacitance. The first calculation unit 11 obtains the absolute value of the impedance indicating the electrical characteristics of the soil 100a as the first information by using the AC voltage and the AC current. The absolute value of the impedance has a predetermined relationship with the phase of impedance. The second calculation unit 12 obtains the phase of the impedance by calculating the absolute value of the impedance. The third calculation unit 13 obtains an evaluation value indicating the characteristics of the soil 100a by using the absolute value of the impedance and the phase of the impedance. Of the two parameters required to obtain the evaluation value, the phase of the impedance is obtained by calculation rather than measurement. Therefore, the measured value for obtaining the evaluation value of the soil 100a is only the absolute value of the impedance. The absolute value of the impedance can be averaged. According to the averaging process, it is possible to reduce a decrease in measurement accuracy. As a result, the soil evaluation sensor 1 can suppress the influence of the measurement accuracy included in the measured value for obtaining the evaluation value of the soil. In addition, the soil evaluation sensor 1 can obtain a good evaluation value.

Incidentally, in the fields of agriculture and disaster prevention, it is desired to expand the measurable region of a sensor. In particular, it is desired to expand the measurable region of a sensor that measures the water content. As the current sensor for measuring the water content, there are a sensor that adopts a TDR method (Time Domain Reflectometry), a tensiometer, a capacitance type sensor, and the like. The sensor that adopts the TDR method measures the water content using the reflection of electromagnetic waves. The tensiometer measures the negative pressure of water based on the water retention of the soil. The capacitance type sensor measures the water content by detecting the difference in relative permittivity based on electrical impedance.

The electrode shape of these sensors has a predetermined shape. As a result, the measurement direction and the measurable region of these sensors are determined. For example, the measurable region is several mm to several tens of cm. In order to cover a desired measurement region by using these sensors, it is necessary to dispose a plurality of sensors. For example, as such a usage form, there is a form in which the water content of a region including the roots of the plant is mead. In addition, as another usage form, there is a form in which the distribution of the water content on the slope of a mountain is measured in a three-dimensional manner.

When using a plurality of sensors, it is necessary to avoid interference between the sensors. As a result, the sensors adjacent to each other are disposed at predetermined intervals therebetween. According to such an arrangement, an unmeasured region that is not included in the measurement region of any sensor is generated in the measurement target region.

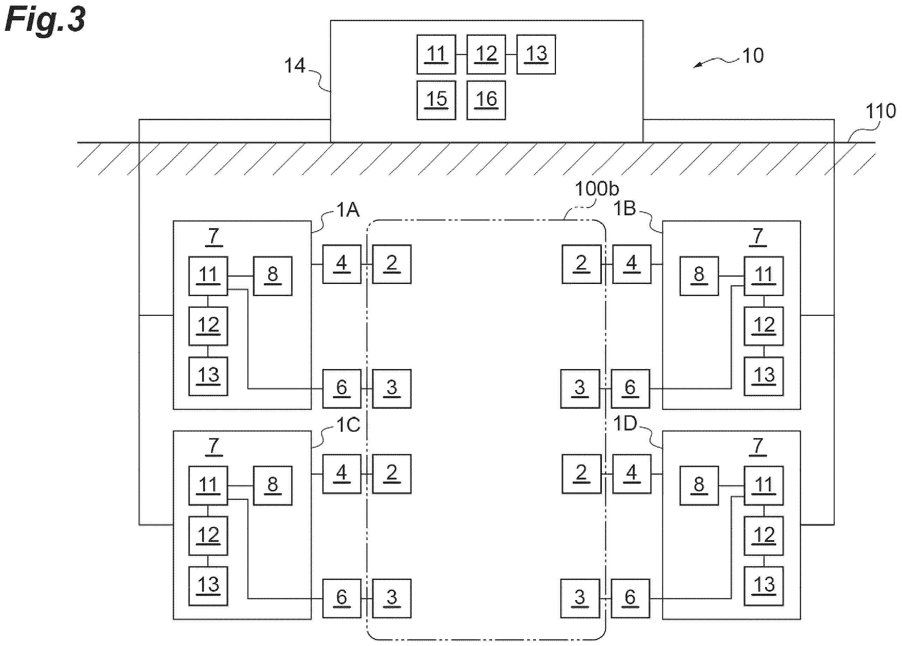

Therefore, a soil evaluation system 10 shown in FIG. 3 can expand the measurement target region without generating an unmeasured region in the measurement target region. That is, the soil evaluation sensors 1A, 1B, 1C, and 1D cooperate with each other to perform measurement in a two-dimensional plane or a three-dimensional space with no gaps. Specifically, in the soil evaluation system 10, the capacitance type soil evaluation sensors 1A, 1B, 1C, and 1D that measure impedance cooperate with each other. As described above, each of the soil evaluation sensors 1A, 1B, 1C, and 1D includes a voltage application circuit (the electrode end 2 and the voltage generation unit 4) and a current detection circuit (the electrode end 3 and the current measurement unit 6). That is, the soil evaluation sensors 1A, 1B, 1C, and 1D are composite circuits. Linking the soil evaluation sensors 1A, 1B, 1C, and 1D to each other means providing an AC voltage from one soil evaluation sensor to the soil 100a and obtaining an AC current due to the AC voltage using another soil evaluation sensor.

As shown in FIG. 3, the soil evaluation system 10 includes the four soil evaluation sensors 1A, 1B, 1C, and 1D and a control unit 14. The number of soil evaluation sensors 1 included in the soil evaluation system 10 may be at least two or more. For example, the number of soil evaluation sensors 1 may be two. In addition, the number of soil evaluation sensors 1 may be five or more. Each soil evaluation sensor 1 is electrically connected to the control unit 14. The connection between the soil evaluation sensor 1 and the control unit 14 may be wired. In addition, the connection between the soil evaluation sensor 1 and the control unit 14 may be wireless. The single unit configuration of the soil evaluation sensors 1A, 1B, 1C, and 1D is the same as that of the soil evaluation sensor 1 of the first embodiment. Therefore, a detailed description of the single unit configuration will be omitted.

The soil evaluation sensors 1A, 1B, 1C, and 1D may function as a first sensor unit or a second sensor unit depending on the mode of use thereof. The electrode ends 2 and 3 provided in each of the soil evaluation sensors 1A, 1B, 1C, and 1D function as a first electrode portion or a second electrode portion depending on the mode of use thereof. The voltage generation unit 4 provided in each of the soil evaluation sensors 1A, 1B, 1C, and 1D functions as a first voltage generation unit or a second voltage generation unit depending on the usage mode thereof. The current measurement unit 6 provided in each of the soil evaluation sensors 1A, 1B, 1C, and 1D functions as a first current measurement unit or a second current measurement unit depending on the usage mode thereof. A switch circuit 21 provided in each of the soil evaluation sensors 1A, 1B, 1C, and 1D functions as a first switching unit or a second switching unit depending on the usage mode thereof.

The control unit 14 includes the first calculation unit 11, the second calculation unit 12, the third calculation unit 13, a sensor control unit 15, and a display processing unit 16.

The sensor control unit 15 generates a control signal for controlling the operation of the soil evaluation sensor 1. In addition, the sensor control unit 15 provides a control signal to each of the soil evaluation sensors 1A, 1B, 1C, and 1D. The control signal determines the soil evaluation sensors 1A, 1B, 1C, and 1D that provides an AC current to the soil 100a and the soil evaluation sensor 1 that obtains an AC current from the soil 100a.

The first calculation unit 11, the second calculation unit 12, and the third calculation unit 13 are the same as those provided in the soil evaluation sensor 1 as described previously. Therefore, a detailed description thereof will be omitted.

In the example shown in FIG. 3, a region 100b of the soil surrounded by the two-dot chain line is a measurement target (measurement target region). This region 100b is larger than the measurement range R1 of each of the soil evaluation sensors 1A, 1B, 1C, and 1D. The soil evaluation sensors 1A, 1B, 1C, and 1D may be disposed so as to surround the region 100b. For example, the shape of the region 100b is a two-dimensional rectangular shape. A pair of soil evaluation sensors 1A and 1B may be disposed so as to interpose the upper part of the region 100b therebetween. Another pair of soil evaluation sensors 1C and 1D may be disposed so as to interpose the lower part of the region 100b therebetween.

The soil evaluation sensor 1 may be disposed at a position where the measurement ranges R1 of the respective soil evaluation sensors 1A, 1B, 1C, and 1D do not overlap each other. For example, the distance from the soil evaluation sensor 1A to the soil evaluation sensor 1B is at least twice the length indicating the measurement range R1 of the soil evaluation sensor 1A.

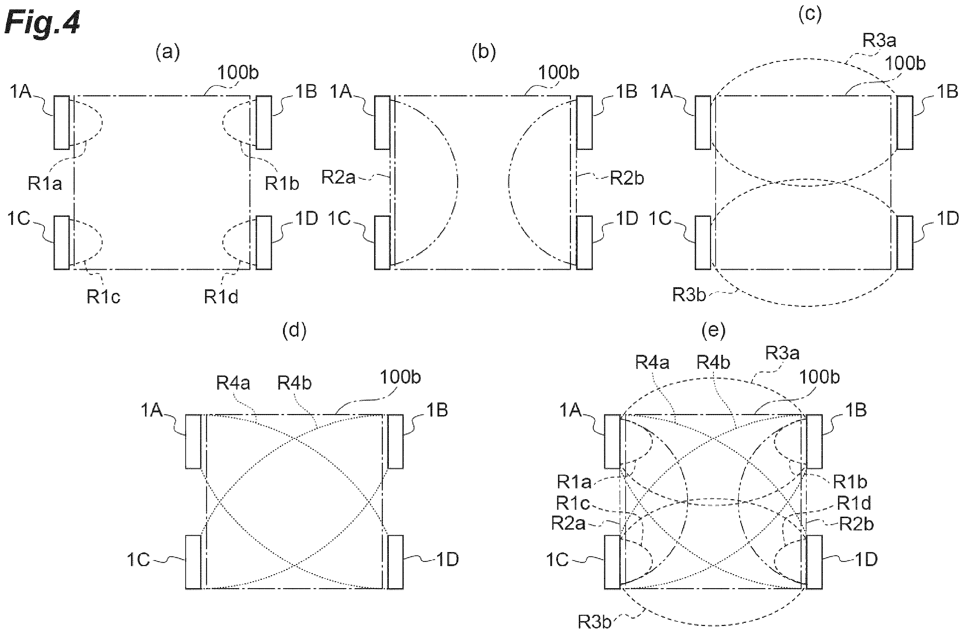

According to such an arrangement, measurement ranges R1a, R1b, R1c, and R1d shown in FIG. 4(a) are formed by the respective soil evaluation sensors 1A, 1B, 1C, and 1D. By making the soil evaluation sensors 1A, 1B, 1C, and 1D cooperate with each other, measurement ranges R2a and R2b shown in FIG. 4(b) are formed. In addition, measurement ranges R3a and R3b shown in FIG. 4(c) and measurement ranges R4a and R4b shown in FIG. 4(d) are formed. As shown in FIG. 4(e), these measurement ranges R1a, R1b, R1c, R1d, R2a, R2b, R3a, R3b, R4a, and R4b overlap each other. Therefore, in the region 100b, there is no unmeasured region that is not included in the measurement ranges R1a, R1b, R1c, R1d, R2a, R2b, R3a, R3b, R4a, and R4b.

The measurement ranges R1a, R1b, R1c, R1d, R2a, R2b, R3a, R3b, R4a, and R4b are formed by operating the soil evaluation sensor 1 based on the control signal provided by the control unit 14.

The measurement ranges R1a, R1b, R1c, and R1d shown in FIG. .4(a) are formed by the respective soil evaluation sensors 1A, 1B, 1C, and 1D. In this configuration, the respective soil evaluation sensors 1A, 1B, 1C, and 1D do not cooperate with each other.

The sensor control unit 15 operates the soil evaluation sensors 1A, 1B, 1C, and 1D so as to provide an AC current to the soil 100a. The sensor control unit 15 measures the AC current in each of the soil evaluation sensors 1A, 1B, 1C, and 1D. That is, in this control mode, the respective soil evaluation sensors 1A, 1B, 1C, and 1D operate independently.

The measurement range R2a shown in FIG. 4(b) is formed by the soil evaluation sensors 1A and 1C in cooperation with each other. The measurement range R2b is formed by the soil evaluation sensors 1B and 1D in cooperation with each other. The measurement ranges R2a and R2b are formed by the soil evaluation sensors 1A and 1C adjacent to each other in the vertical direction (up-and-down direction) and the soil evaluation sensors 1B and 1D adjacent to each other in the vertical direction (up-and-down direction), respectively.

The sensor control unit 15 causes the soil evaluation sensor 1A to provide an AC voltage to the soil 100a. The sensor control unit 15 obtains an AC voltage from the current measurement unit 6 of the soil evaluation sensor 1C. The control unit 14 obtains an evaluation value by using the information regarding the AC voltage. With this configuration, it is possible to obtain an evaluation value for the soil 100a included in the measurement range R2a. The sensor control unit 15 causes the soil evaluation sensor 1B to provide an AC voltage to the soil 100a. The sensor control unit 15 obtains an AC current from the current measurement unit 6 of the soil evaluation sensor 1D. The control unit 14 obtains an evaluation value by using the information regarding the AC voltage. With this configuration, it is possible to obtain an evaluation value for the soil 100a included in the measurement range R2b.

The measurement range R3a shown in FIG. 4(c) is formed by the soil evaluation sensors 1A and 1B in cooperation with each other. The measurement range R3b is formed by the soil evaluation sensors 1C and 1D in cooperation with each other. That is, the measurement ranges R3a and R3b are formed by the soil evaluation sensors 1A and 1B adjacent to each other in the horizontal direction (left and right direction) and the soil evaluation sensors 1C and 1D adjacent to each other in the horizontal direction (left and right direction), respectively.

The sensor control unit 15 causes the soil evaluation sensor 1A to provide an AC voltage to the soil 100a. The sensor control unit 15 obtains an AC current from the current measurement unit 6 of the soil evaluation sensor 1B. The control unit 14 obtains an evaluation value by using the information regarding the AC voltage. With this configuration, it is possible to obtain an evaluation value for the soil 100a included in the measurement range R3a. The sensor control unit 15 causes the soil evaluation sensor 1C to provide an AC voltage to the soil 100a. The sensor control unit 15 obtains an AC current from the current measurement unit 6 of the soil evaluation sensor 1D. The control unit 14 obtains an evaluation value by using the information regarding the AC voltage. With this configuration, it is possible to obtain an evaluation value for the soil 100a included in the measurement range R3b.

The measurement range R4a shown in FIG. 4(d) is formed by the soil evaluation sensors 1A and 1D in cooperation with each other. The measurement range R4b is formed by the soil evaluation sensors 1B and 1C in cooperation with each other. That is, the measurement ranges R4a and R4b are formed by the soil evaluation sensors 1A and 1D adjacent to each other in the oblique direction and the soil evaluation sensors 1B and 1C adjacent to each other in the oblique direction, respectively.

The sensor control unit 15 causes the soil evaluation sensor 1A to provide an AC voltage to the soil 100a. The sensor control unit 15 obtains an AC voltage from the current measurement unit 6 of the soil evaluation sensor 1D. The control unit 14 obtains an evaluation value by using the information regarding the AC voltage. With this configuration, it is possible to obtain an evaluation value for the soil 100a included in the measurement range R4a. The sensor control unit 15 causes the soil evaluation sensor 1B to provide an AC voltage to the soil 100a. The sensor control unit 15 obtains an AC current from the current measurement unit 6 of the soil evaluation sensor 1C. The control unit 14 obtains an evaluation value by using the information regarding the AC voltage. With this configuration, it is possible to obtain an evaluation value for the soil 100a included in the measurement range R4b.

Measurement operations in the measurement ranges R1a, R1b, R1c, and R1d, measurement operations in the measurement ranges R2a and R2b, measurement operations in the measurement ranges R3a and R3b, and measurement operations in the measurement ranges R4a and R4b are performed in a time-division manner. Specifically, first, the sensor control unit 15 performs measurement operations in the measurement ranges R1a, R1b, R1c, and R1d. More specifically, the sensor control unit 15 performs a measurement operation in the measurement range R1a, then performs a measurement operation in the measurement range R1b, then performs a measurement operation in the measurement range R1c, and finally performs a measurement operation in the measurement range R1d. That is, the periods of the operations for the measurement ranges R1a, R1b, R1c, and R1d do not overlap with each other. Such processing is referred to as time division processing. Then, the sensor control unit 15 performs measurement operations in the measurement ranges R2a and R2b as time division processing. Subsequently, the sensor control unit 15 performs measurement operations in the measurement ranges R3a and R3b as time division processing. Then, the sensor control unit 15 performs measurement operations in the measurement ranges R4a and R4b as time division processing. In addition, in the measurement operations using time division, it is desirable to perform measurement between one-to-one sensors at the same time in order to avoid interference between the operating sensors. However, in order to shorten the measurement time, if interference between sensors is not a problem, measurement may be performed simultaneously between a plurality of pairs of sensors. For example, the measurement operation in the measurement range R1a and the measurement operation in the measurement range R1b may be performed in parallel.

The display processing unit 16 of the control unit 14 calculates the distribution of the evaluation values in the region 100b using the evaluation values obtained corresponding to the measurement ranges R1a, R1b, R1c, R1d, R2a, R2b, R3a, R3b, R4a, and R4b. As a result, for example, it is possible to obtain information regarding the two-dimensional distribution of the water content in the region 100b.

Incidentally, when different soil evaluation sensors 1A, 1B, 1C, and 1D are operated in cooperation with each other, it is conceivable to use phase information. The phase information indicates the time lag of the AC current due to the AC voltage. When the phase information is used, time synchronization of the soil evaluation sensors 1A, 1B, 1C, and 1D is required. For example, when the soil evaluation sensors 1A and 1B are operated in cooperation with each other to measure the phase information, the time when the soil evaluation sensor 1A starts to provide the AC voltage and the time when the soil evaluation sensor 1B starts to detect the AC current are made to synchronize with each other. In this case, for example, assuming that the frequency of the AC signal is 1 MHz (megahertz), a time resolution of 0.1 nsec (nanoseconds) or less is required. However, time synchronization using GPS lacks accuracy. In addition, a circuit that generates an ultra-high precision clock consumes a large amount of power and occupies a large area. Therefore, the circuit that generates an ultra-high precision clock is not suitable for a sensor for measurement used in the field. In addition, the circuit that generates an ultra-high precision clock often contains noise in the soil during phase detection. Therefore, it is difficult to perform stable measurement in the circuit that generates an ultra-high precision clock.

Therefore, the soil evaluation system 10 of the second embodiment adopts a configuration that does not require time synchronization between the sensors. The soil evaluation system 10 does not acquire information regarding the phase of impedance by measurement. The soil evaluation system 10 obtains the information regarding the phase of impedance from the absolute value of impedance.

The soil evaluation system 10 performs a first operation and a second operation. In the first operation, the soil evaluation sensors 1A, 1B, 1C, and 1D are operated independently. In the second operation, the soil evaluation sensors 1A, 1B, 1C, and 1D are operated in cooperation with each other. As a result, it is possible to generate the measurement ranges R1a, R1b, R1c, and R1d by the first operation and the measurement ranges R2a, R2b, R3a, and R3b by the second operation. Therefore, it is possible to form a measurement range without leakage for a wide region 100b. The control unit 14 obtains the phase of impedance by calculation using the absolute value of the impedance. According to this process, it is not necessary to timely synchronize the timing of providing the AC voltage and the timing of obtaining the AC current at the time of the second operation to operate the soil evaluation sensors 1A, 1B, 1C, and 1D in cooperation with each other. Therefore, a measurement error due to phase measurement does not occur.

As a result, the soil evaluation system 10 can obtain a good evaluation value in the cooperative operation of the soil evaluation sensors 1A, 1B, 1C, and 1D.

From another point of view, in the soil evaluation system 10, the circuit for detecting the absolute value of impedance has no problem in synchronization in units of several milliseconds. The absolute value of impedance can be averaged at the time of measurement. As a result, the mixed random noise can be reduced. According to the inventors of the present application, based on the fact that an electrical equivalent circuit including the soil 100a and water is shown by a parallel circuit of electrical resistance and capacitance, there is a predetermined correspondence between the frequency change of the absolute value of impedance and the phase. As a result, the inventors came up with the idea that the phase of impedance can be calculated from the absolute value of impedance. The inventors have completed a technique for accurately acquiring the value of the capacitance by estimating the phase information based on the plurality of absolute values using the absolute value of two or more types of impedance based on two or more types of frequencies.

In short, the soil evaluation system 10 can measure the water content of the soil 100a, the distribution of water, and the distribution of total ion concentration without gaps without directly measuring the phase information that requires high measurement accuracy. That is, the soil evaluation system 10 calculates the phase by differentiating the absolute value of impedance by frequency. As a result, it is possible to obtain information regarding the capacitance from the absolute value of impedance without requiring accurate time synchronization among a plurality of soil evaluation sensors 1A, 1B, 1C, and 1D. That is, the soil evaluation system 10 enables a wide range of measurement without gaps by making sensors having independent time clocks cooperate with each other.

The soil evaluation system 10 can be applied to, for example, accurate observation of the environment in which the roots of plants are present and detection of a region having high water content on the mountain slope leading to slope collapse. In addition, in the soil evaluation system 10, individual sensors can be freely disposed according to the installation location. Therefore, in the soil evaluation system 10, it is possible to realize a flexible sensor layout according to the installation location. As a result, the soil evaluation system 10 is not limited to the application examples described above, and can be applied to various cases that require evaluation of the characteristics of the soil 100a.

The soil evaluation system 10 does not require highly accurate time synchronization, and it is easy to reduce power consumption. The soil evaluation system 10 can perform stable measurement in the soil 100a having a large disturbance noise. In the soil evaluation system 10, since it is possible to form a measurement region without gaps, a large measurement region can be formed by using a plurality of small soil evaluation sensors 1A, 1B, 1C, and 1D. Then, the soil evaluation sensors 1A, 1B, 1C, and 1D can be flexibly disposed with a high degree of freedom according to the shape of the measurement region.

While the embodiment of the present invention has been described above, the present invention is not limited to the above embodiment.

<First modification example>

In the soil evaluation sensor 1, the electrode end 2 for providing an AC voltage and the electrode end 3 for obtaining an AC current are fixed. In other words, the electrode end 2 had only a function of providing an AC voltage. In addition, the electrode end 3 has only a function of obtaining an AC current. For example, a soil evaluation sensor 1E used in a soil evaluation system of a first modification example may provide an AC voltage to the soil 100a by using both the electrode ends 2 and 3. In addition, the soil evaluation sensor 1E may obtain an AC current from the soil 100a by using both the electrode ends 2 and 3.

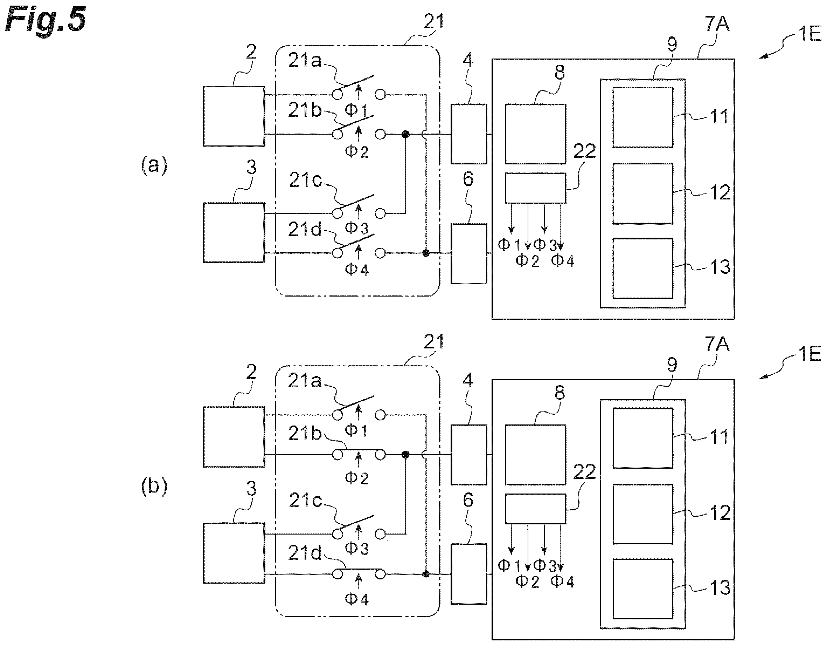

When the soil evaluation sensor 1 is used in the soil evaluation system 10, a configuration in which the voltage generation unit 4 and the current measurement unit 6 are not connected to any of the electrode ends 2 and 3 is also effective. For example, in the soil evaluation system 10, when measurement is performed using the combination of the soil evaluation sensors 1A and 1B, the soil evaluation sensors 1C and 1D are not operated. In this case, in the soil evaluation sensors 1C and 1D, as shown in FIG. 5(a), the switch circuit 21 does not connect the voltage generation unit 4 and the current measurement unit 6 to any of the electrode ends 2 and 3. That is, in the switch circuit 21, all of the switches 21a, 21b, 21c, and 21d are disconnected. According to this operation, the current flowing through the soil does not flow into the soil evaluation sensors 1C and 1D. Therefore, the current flowing through the soil flows into either one of the soil evaluation sensors 1A and 1B on the detection side. As a result, the measurement using the soil evaluation sensors 1A and 1B can be performed satisfactorily. In this form, the soil evaluation sensor 1A may use only the electrode end 2 to provide the AC voltage. In the switch circuit 21, the switch 21b is connected. In addition, in the switch circuit 21, the switches 21a, 21c, and 21d are disconnected. The soil evaluation sensor 1B may use only the electrode end 3 to acquire the AC current. In the switch circuit 21, the switch 21a is connected. In addition, in the switch circuit 21, the switches 21b, 21c, and 21d are disconnected.

As shown in FIG. 5(a), the soil evaluation sensor 1E includes the electrode ends 2 and 3, the switch circuit 21, the voltage generation unit 4, the current measurement unit 6, and a processing unit 7A. The switch circuit 21 is disposed between the electrode ends 2 and 3 and the voltage generation unit 4. In addition, the switch circuit 21 is also disposed between the electrode ends 2 and 3 and the current measurement unit 6.

The switch circuit 21 includes the four switches 21a, 21b, 21c, and 21d. These switches 21a, 21b, 21c, and 21d switch between a connection state and a non-connection state (disconnection state) according to control signals $\phi1$, $\phi2$, $\phi3$, and $\phi4$ provided from a switch control unit 22 of the processing unit 7A. The switch 21a is disposed between the electrode end 2 and the current measurement unit 6. The switch 21a switches the state between the electrode end 2 and the current measurement unit 6 to a connection state or a disconnection state. The switch 21b is disposed between the electrode end 2 and the voltage generation unit 4. The switch 21b switches the state between the electrode end 2 and the voltage generation unit 4 to a connection state or a disconnection state. The switch 21c is disposed between the electrode end 3 and the voltage generation unit 4. The switch 21c switches the state between the electrode end 3 and the voltage generation unit 4 to a connection state or a disconnection state. The switch 21d is disposed between the electrode end 3 and the current measurement unit 6. The switch 21d switches the state between the electrode end 3 and the current measurement unit 6 to a connection state or a disconnection state.

The processing unit 7A further includes the switch control unit 22 in addition to the voltage control unit 8 and the evaluation value calculation unit 9. The switch control unit 22 controls the switch circuit 21 so as to have three different connection modes. The switch circuit 21 operates based on a control signal provided from the control unit 14. The switch circuit 21 may be directly controlled by the control signal provided from the control unit 14.

The switch control unit 22 controls the switch circuit 21 so as to have a connection mode shown in FIG. 5(b) as a first mode. In the first mode, the soil evaluation sensor 1E as a single unit obtains an evaluation value. Specifically, the switch control unit 22 controls the switch circuit 21 so that the switches 21b and 21d are connected and the switches 21a and 21c are not connected. As a result, the electrode end 2 is connected to the voltage generation unit 4. The electrode end 3 is connected to the current measurement unit 6.

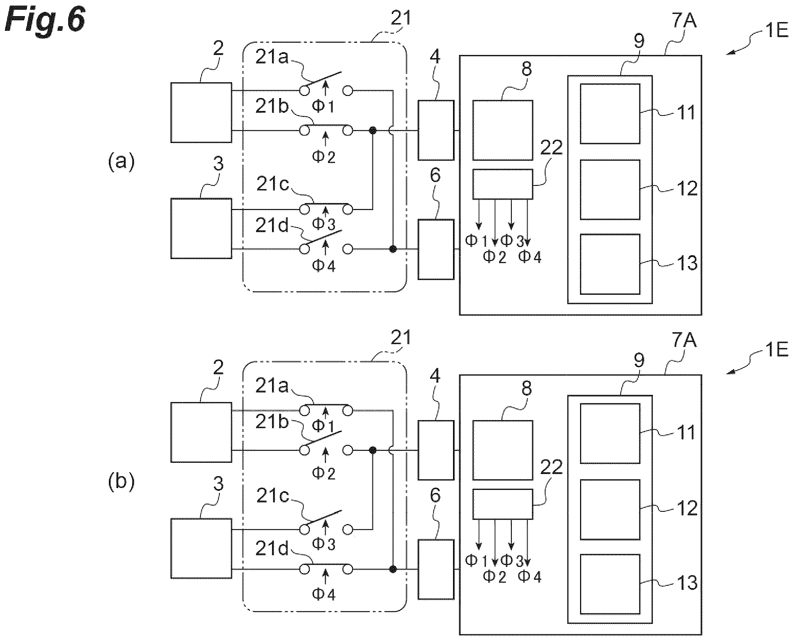

The switch control unit 22 controls the switch circuit 21 so as to have a connection mode shown in FIG. 6(a) as a second mode. In the second mode, the soil evaluation sensor 1E as a single unit provides an AC voltage. Specifically, the switch control unit 22 controls the switch circuit 21 so that the switches 21b and 21c are connected and the switches 21a and 21d are not connected. As a result, both the electrode ends 2 and 3 are connected to the voltage generation unit 4. That is, the current measurement unit 6 is not connected to any of the electrode ends 3.

The switch control unit 22 controls the switch circuit 21 so as to have a connection mode shown in FIG. 6(b) as a third mode. In the third mode, the soil evaluation sensor 1E as a single unit obtains an AC current. Specifically, the switch control unit 22 controls the switch circuit 21 so that the switches 21a and 21d are connected and the switches 21b and 21c are not connected. As a result, both the electrode ends 2 and 3 are connected to the current measurement unit 6. That is, the voltage generation unit 4 is not connected to any of the electrode ends 2.

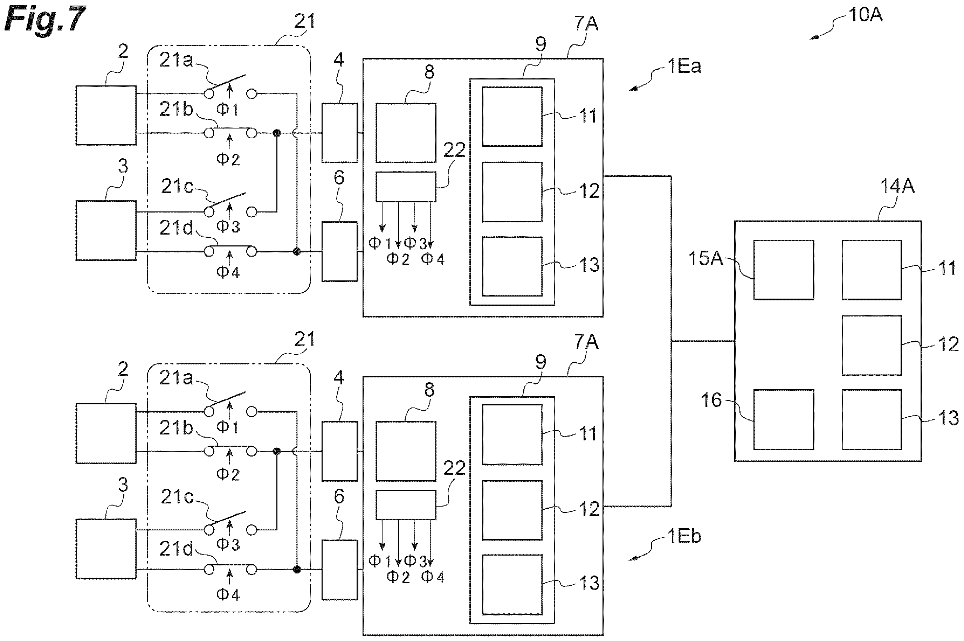

As shown in FIG. 7, a soil evaluation system 10A of a modification example includes soil evaluation sensors 1Ea and 1Eb and a control unit 14A. The control unit 14A includes a sensor control unit 15A that provides a control signal to the switch control unit 22. The sensor control unit 15A determines a sensor that provides an AC voltage, and outputs a control signal for connecting the voltage generation unit 4 to the electrode ends 2 and 3 in the sensor. In addition, the sensor control unit 15A determines a sensor that receives an AC current, and outputs a control signal for connecting the current measurement unit 6 to the electrode ends 2 and 3 in the sensor.

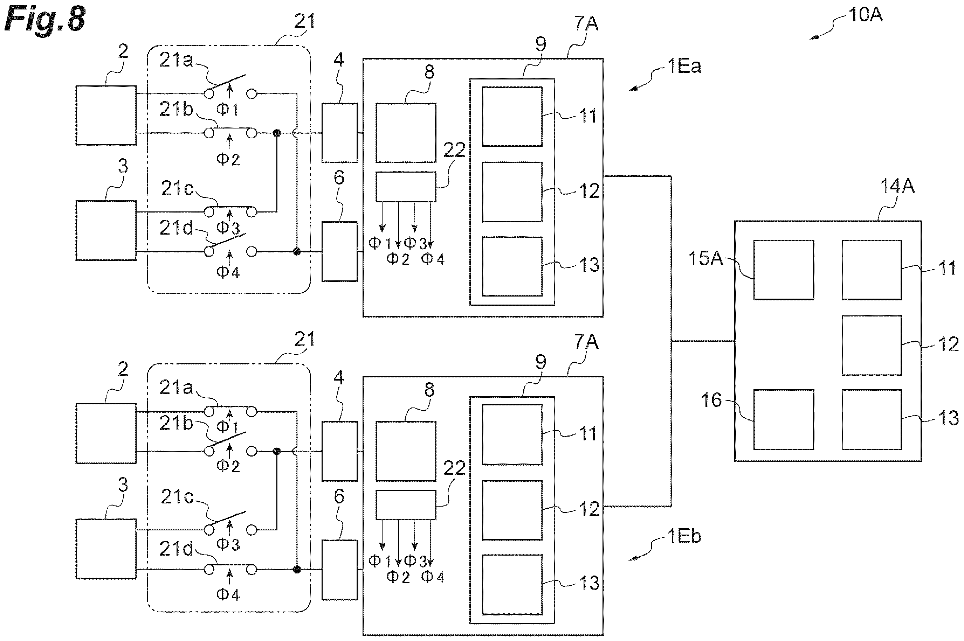

For example, as shown in FIG. 7, the control unit 14A controls the switch circuits 21 of the soil evaluation sensors 1Ea and 1Eb to be in the first mode. As a result, the control unit 14A can be configured to obtain the evaluation value by the individual soil evaluation sensors 1Ea and 1Eb. On the other hand, as shown in FIG. 8, the control unit 14A controls the switch circuit 21 of the soil evaluation sensor 1Ea to be in the second mode and the switch circuit 21 of the soil evaluation sensor 1Eb to be in the third mode. As a result, the soil evaluation sensors 1Ea and 1Eb can be configured to cooperate with each other.

The soil evaluation system 10A of the first modification example provides an AC voltage from the two electrode ends 2 and 3 to the soil 100a. As a result, the soil evaluation system 10A can satisfactorily provide an AC voltage to the soil 100a. In addition, the soil evaluation system 10A obtains an AC current from the soil 100a using the two electrode ends 2 and 3. As a result, the soil evaluation system 10A can accurately obtain the AC current. Therefore, the soil evaluation system 10A can obtain the water content and the total ion concentration as good evaluation values.

<Second modification example>

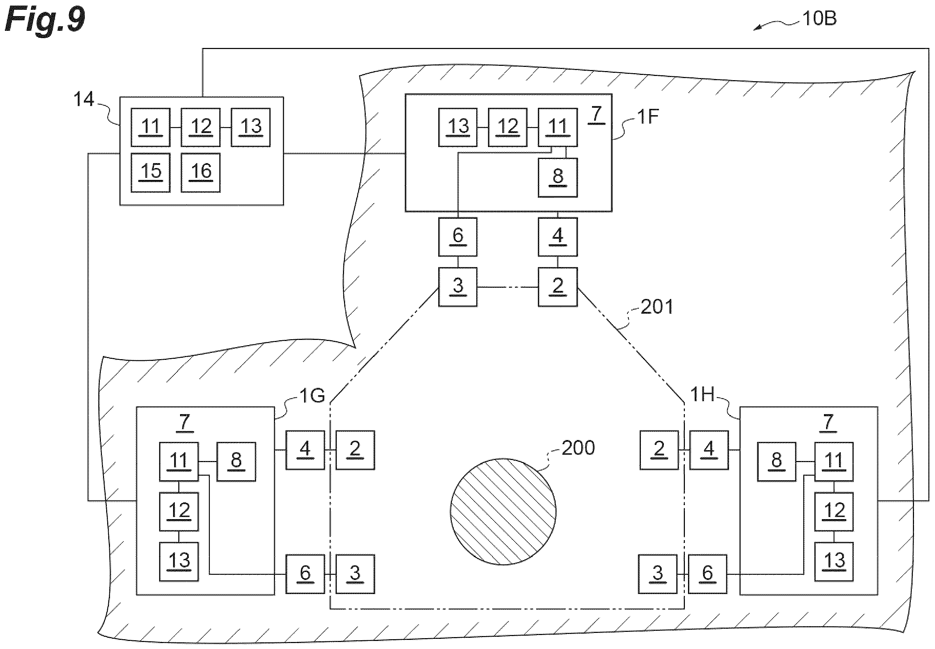

The above-described soil evaluation system 10 sets a measurement target region which is a virtual plane perpendicular to the horizontal direction. Then, a plurality of soil evaluation sensors 1A, 1B, 1C, and 1D configuring the soil evaluation system 10 are disposed so as to be included in the virtual plane. For example, the virtual plane as a measurement target region may be set in a desired orientation. That is, the measurement target region may be set as a virtual plane perpendicular to the vertical direction. In addition, the measurement target region is not limited to the two-dimensional plane. The measurement target region may be a three-dimensional region. FIG. 9 shows a soil evaluation system 10B when the measurement target region is a virtual plane perpendicular to the vertical direction. Such a configuration can be appropriately applied to, for example, evaluation of the water content in a region including the root 200 of a plant. As shown in FIG. 9, the soil evaluation system 10B sets an approximately triangular region 201 including the root 200, and includes three soil evaluation sensors 1F, 1G, and 1H disposed so as to surround the region 201. According to such an arrangement, it is not necessary to dispose the soil evaluation sensors 1F, 1G, and 1H directly in the root 200. Therefore, it is possible to measure the water content and the like without interfering with the region 201 that the user really desires to measure.

The soil evaluation system 10 described above operates in a one-to-one manner as a combination of a plurality of soil evaluation sensors 1A, 1B, 1C, and 1D. That is, for one soil evaluation sensor that provides an AC voltage, there is one soil evaluation sensor that obtains an AC current due to the AC voltage. For example, an AC voltage may be provided from one soil evaluation sensor, and an AC current due to the AC voltage may be obtained by a plurality of soil evaluation sensors. For example, the soil evaluation sensor 1A may provide an AC voltage, and the soil evaluation sensors 1B, 1C, and 1D may obtain an AC current. According to such an operation, it is possible to shorten the measurement time.

<Third modification example>

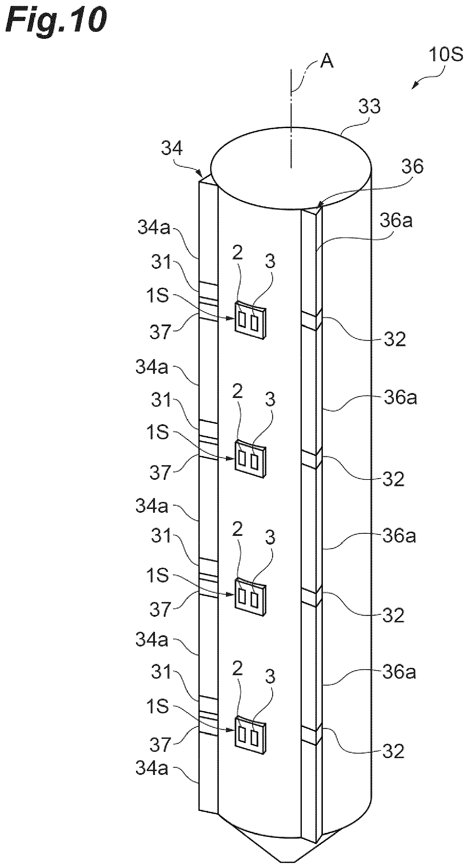

The above-described soil evaluation sensor 1 is exemplified as a sensor chip integrated on the semiconductor substrate. In this case, the electrode ends 2 and 3 are provided on the main surface of the substrate of the sensor chip. That is, the electrode ends 2 and 3 are in two-dimensional contact with the soil 100a. As shown in FIG. 10, a soil evaluation sensor 1S configuring a soil evaluation system 10S of a third modification example has extension electrodes 31 and 32. FIG. 10 shows the soil evaluation system 10S of the third modification example. The measurement target region of the soil evaluation system 10S is a cylindrical region set with the vertical direction as the axis A. Therefore, in the soil evaluation system 10S, two soil evaluation sensors 1S are disposed around the axis A. In addition, in the soil evaluation system 10S, four sets of units each of which is configured to include two soil evaluation sensors 1S disposed around the axis A are disposed at equal intervals along the axis A. For example, the soil evaluation system 10S includes a tubular main body 33, ridges 34 and 36 (first and second protruding portions), and the soil evaluation sensor 1S.

The main body 33 is a base portion to which the soil evaluation sensor 1S is fixed. The shape of the distal end portion of the main body 33 is conical. According to this shape, the soil evaluation system 10S can be easily inserted into the soil 100a. A plurality of soil evaluation sensors 1S are provided on the outer peripheral surface of the main body 33. The arrangement of the soil evaluation sensors 1S is as described above.

In the soil evaluation sensor 1S, either the electrode end 2 or the extension electrode 31 may be arbitrarily selected as an electrode for providing the AC voltage. Similarly, in the soil evaluation sensor 1S, either the electrode end 3 or the extension electrode 32 may be arbitrarily selected as an electrode for acquiring the AC current. The soil evaluation sensor 1S may provide an AC voltage using both the electrode end 2 and the extension electrode 31.

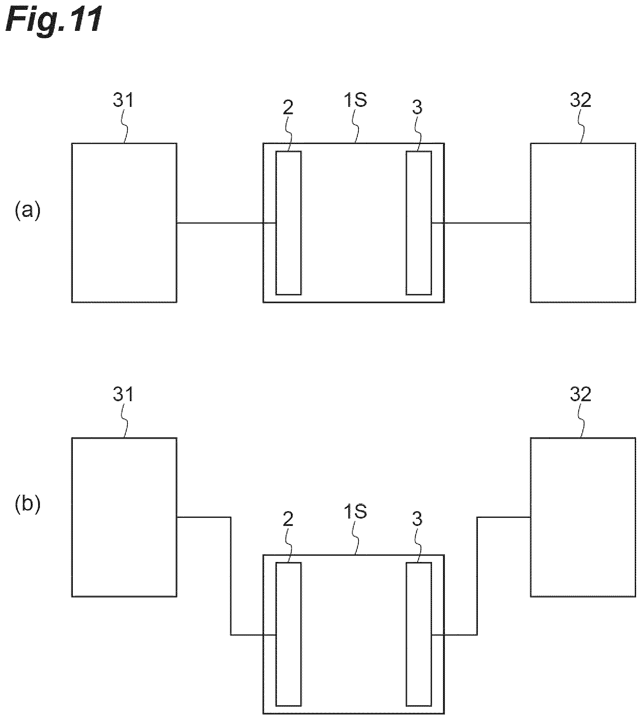

For example, when performing measurement using the electrode ends 2 and 3 and measurement using the extension electrodes 31 and 32, the soil evaluation sensor 1S is disposed between the extension electrodes 31 and 32 (see FIGS. 11(a) and 11(b)). In addition, between the extension electrodes 31 and 32, as shown in FIG. 11(a), the soil evaluation sensor 1S is disposed on a virtual straight line connecting the extension electrode 31 and the extension electrode 32 to each other. In addition, between the extension electrodes 31 and 32, as shown in FIG. 11(b), the soil evaluation sensor 1S is not disposed on the virtual straight line connecting the extension electrode 31 and the extension electrode 32 to each other. With such a configuration, it is possible to apply two types of voltages, one in the horizontal direction and the other in the vertical direction. As a result, it is possible to further improve the contact with the soil.

On the other hand, for example, the measurement using the electrode ends 2 and 3 may not be performed, and only the measurement using the extension electrodes 31 and 32 may be performed. In this case, the position of the soil evaluation sensor 1S with respect to the extension electrodes 31 and 32 is not particularly limited.

In addition, the ridges 34 and 36 are provided on the outer peripheral surface of the main body 33 at equal intervals around the axis A. The ridges 34 and 36, which are protruding portions, protrude from the outer peripheral surface of the main body 33. The ridges 34 and 36 extend in the direction of the axis A. The outer peripheral surface of the main body 33 may be regarded as the main surface of the substrate provided in the soil evaluation sensor 1S. The cross-sectional shape of each of the ridges 34 and 36 in the direction of the axis A is, for example, a triangle. The ridge 34 includes an insulating portion 34a, the extension electrode 31 (first extension electrode), and an additional electrode 37. The additional electrode 37 is provided as needed to provide a desired function. For example, the additional electrode 37 may be used to remove static electricity. These components are disposed alternately and at equal intervals along the direction of the axis A. Therefore, the extension electrodes 31 are insulated from each other by the insulating portions 34a having an electrically insulating property. The cross-sectional shapes of the insulating portion 34a, the extension electrode 31, and the additional electrode 37 are the same as each other. Therefore, there is no substantial step between the surface of the insulating portion 34a, the surface of the extension electrode 31, and the surface of the additional electrode 37. As a result, the extension electrode 31 can be easily inserted into the soil 100a. The ridge 36 includes an insulating portion 36a and the extension electrode 32 (second extension electrode). The insulating portion 36a and the extension electrode 32 are disposed at equal intervals in this order.

The electrode surface of the extension electrode 31 is electrically connected to the electrode end 2. The electrode surface of the extension electrode 32 is electrically connected to the electrode end 3. The normal directions of the electrode surfaces are different from each other. The electrode surfaces face different directions. The extension electrode 31 has a three-dimensional structure, not just a two-dimensional plane.

According to such a configuration, an AC voltage is provided from the extension electrode 31 to the soil 100a. An AC current is obtained by the extension electrode 32. The extension electrodes 31 and 32 are in three-dimensional contact with the soil 100a. As a result, good electrical contact with the granular soil 100a can be obtained. Therefore, the AC voltage can be stably provided to the soil 100a. In addition, the AC current can be stably obtained from the soil 100a.

According to the arrangement of the soil evaluation sensor 1S and the extension electrodes 31 and 32 shown in FIG. 11(b), three-dimensional measurement is possible. Specifically, the position of the electrode end 3 with respect to the extension electrode 31 is different in the direction of the axis A from the position of the extension electrode 32 with respect to the extension electrode 31. When the axis A is parallel to the vertical direction, the position of the electrode end 3 is different in height from the position of the extension electrode 32. Then, the direction of the current from the extension electrode 31 to the electrode end 3 is different from the direction of the current from the extension electrode 31 to the extension electrode 32. Therefore, three-dimensional measurement is possible.

<Fourth modification example>

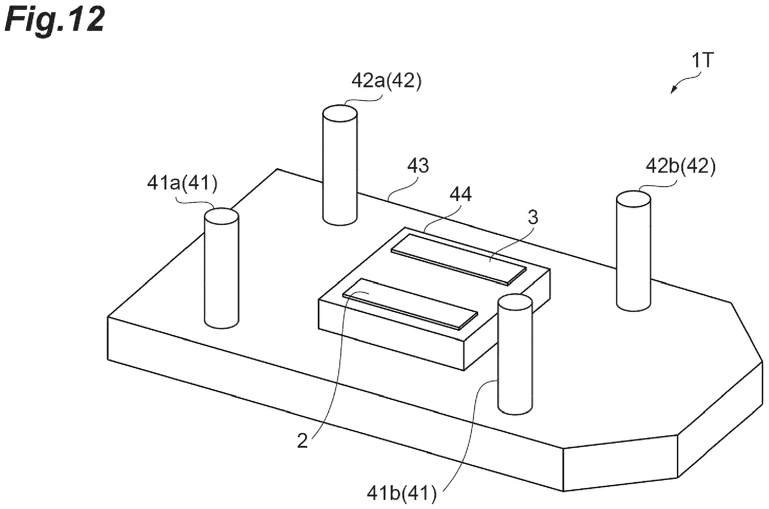

For example, a soil evaluation sensor 1T shown in FIG. 12 may include extension electrodes 41 and 42 having a shape different from that in the third modification example. The soil evaluation sensor 1T has a main body 43, a sensor chip 44 provided with electrode ends 2 and 3, and the extension electrodes 41 and 42 (first and second extension electrodes). The main body 43 is a base of the soil evaluation sensor 1T. The sensor chip 44 is disposed on the main surface of the main body 43. The sensor chip 44 includes functional components, such as the electrode ends 2 and 3, the voltage generation unit 4, the current measurement unit 6, and the processing unit 7.

The extension electrodes 41 and 42 are further erected on the main surface of the main body 43 where the sensor chip 44 is provided. The extension electrodes 41 and 42 are columnar members extending in the normal direction of the main surface. The extension electrodes 41 and 42 are formed of a metallic conductive material. The protruding portion and the extension electrode may be configured as separate members. In addition, the protruding portion and the extension electrode may be integrally configured like the extension electrodes 41 and 42. The extension electrode 41 includes a pair of electrode rods 41a and 41b. The electrode rods 41a and 41b are spaced apart from each other in a direction perpendicular to the normal direction. The electrode rods 41a and 41b of the extension electrode 41 are electrically connected to the electrode end 2. Therefore, the AC voltage is provided from the electrode rods 41a and 41b to the soil 100a. The number of electrode rods included in the extension electrode 41 is not limited to two. The number of electrode rods may be one. The number of electrode rods may be three or more. The extension electrode 41 is not limited to the columnar rod-shaped member. For example, the extension electrode 41 may be a flat plate-shaped member or a rectangular parallelepiped-shaped member that stands up from the main body 43. In addition, in the soil evaluation sensor 1T, a conductive material having the functions of the electrode ends 2 and 3 may be adopted instead of the sensor chip 44.

The extension electrode 42 includes a pair of electrode rods 42a and 42b. The electrode rods 42a and 42b are spaced apart from each other in a direction perpendicular to the normal direction. The electrode rods 42a and 42b are also spaced apart from the electrode rods 41a and 41b. For example, when the soil evaluation sensor 1T is viewed in a plan view, the electrode rods 41a, 41b, 42a, and 42b are disposed at the tops of virtual rectangular regions surrounding the sensor chip 44, respectively. The electrode rods 41a and 41b of the extension electrode 41 are electrically connected to the electrode end 2. Therefore, the AC voltage is provided from the electrode rods 41a and 41b to the soil 100a.

Even with such a soil evaluation sensor 1T, it is possible to make good electrical contact with the granular soil 100a. As a result, the soil evaluation sensor 1T can stably provide an AC voltage to the soil 100a. The soil evaluation sensor 1T can stably obtain an AC current from the soil 100a. In addition, in the region surrounded by the four electrode rods 41a, 41b, 42a, and 42b, the movement of the soil 100a is inhibited. In other words, the soil 100a present in the region surrounded by the electrode rods 41a, 41b, 42a, and 42b is difficult to move. Therefore, the state of the measurement target region is stabilized. As a result, the soil evaluation sensor 1T can obtain a better evaluation value.

<Fifth modification example>

In the soil evaluation system 10 of the embodiment described above, for example, the operation of causing the soil evaluation sensors 1A to 1D not to cooperate with each other as shown in FIG. 4(a) and the operation of causing the soil evaluation sensors 1A to 1D to cooperate with each other as shown in FIGS. 4(b), 4(c), and 4(d) are performed. For example, in a soil evaluation system of a fifth modification example, the operation of causing the soil evaluation sensors 1A to 1D not to cooperate with each other may be omitted, and only the operation of causing the soil evaluation sensors 1A to 1D to cooperate with each other may be performed.

<Sixth modification example>

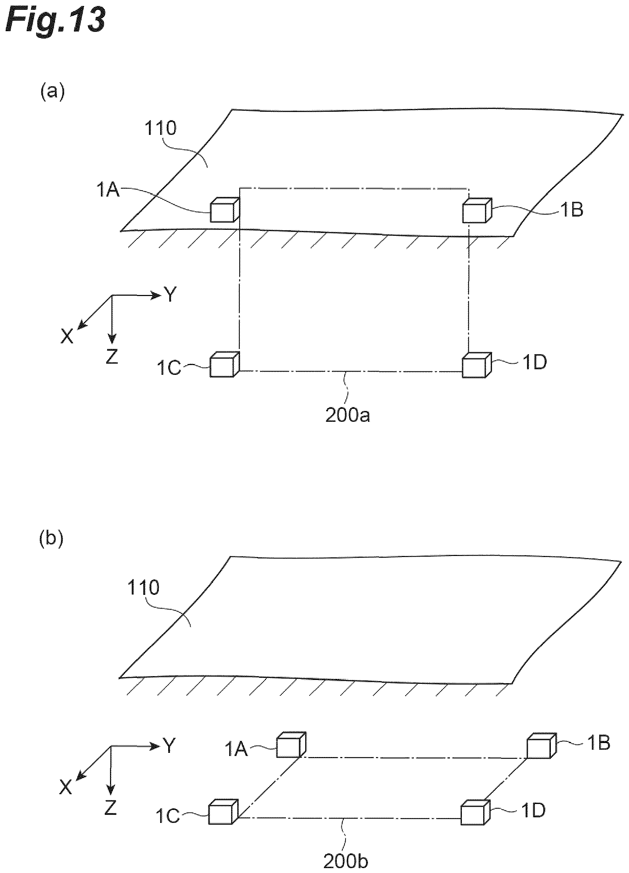

The measurement target region may be set as a two-dimensional surface. Then, the orientation of the surface, which is a measurement target region, may be set to a desired orientation. For example, as shown in FIG. 13(a), the orientation of a measurement target region 200a (target region) may be parallel to the horizontal direction (X axis and Y axis). That is, the measurement target region 200a may cross the ground 110. In addition, as shown in FIG. 13(b), the orientation of a measurement target region 200b may be parallel to the vertical direction (Z axis). That is, the measurement target region 200b may be parallel to the ground 110. In addition, the orientation of the measurement target region may be inclined with respect to the X axis, the Y axis, or the Z axis.

<Seventh modification example>

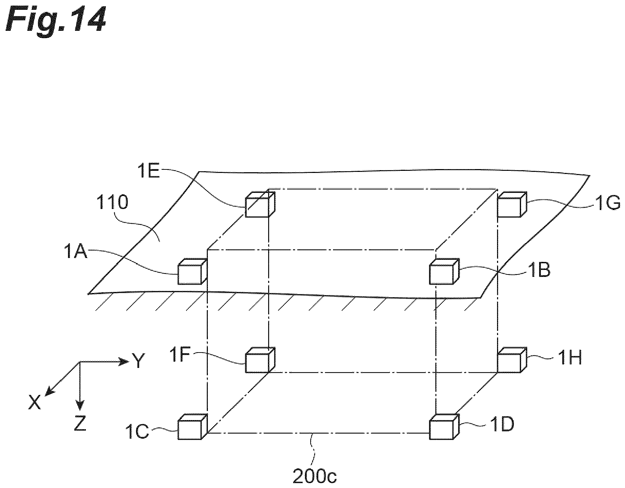

In addition, as shown in FIG. 14, a measurement target region 200c may be set as a three-dimensional region. In this case, soil evaluation sensors 1A to 1H are disposed in the vicinity of the corners of the measurement target region 200c defined as a rectangular parallelepiped. For example, an AC voltage is provided from the soil evaluation sensor 1A, and an AC current is obtained by using some or all of the other soil evaluation sensors 1B to 1H. By such a cooperative operation, the distribution of the water content in the region surrounded by the soil evaluation sensors 1A to 1H can be obtained.

<Eighth modification example>

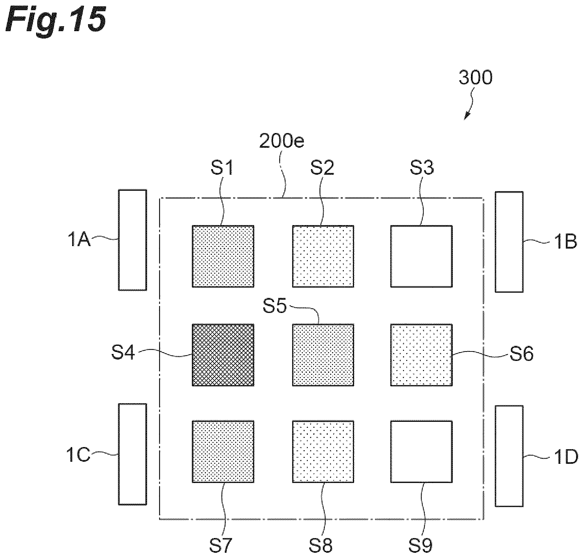

For example, the display processing unit 16 may present an evaluation result as a map 300 as shown in FIG. 15. The map 300 shows the distribution of the water content in a measurement target region 200e. The measurement target region 200e includes a plurality of segments S1 to S9(a plurality of partial regions). Display values (total calculation values) are given to the segments S1 to S9. These display values may be evaluation values themselves output from the third calculation unit 13, or may be obtained by performing numerical processing on the evaluation values. The display processing unit 16 may display a specific display value (numerical value) given to each of the segments S1 to S9. In addition, the display processing unit 16 may perform visual display by a contour diagram or the like shown by using a plurality of colors or shades associated with the display values.

Hereinafter, an example of a method of obtaining a display value from the evaluation value will be described. In this explanation, it is assumed that the evaluation value obtained by the operation shown in FIG. 4 is used.

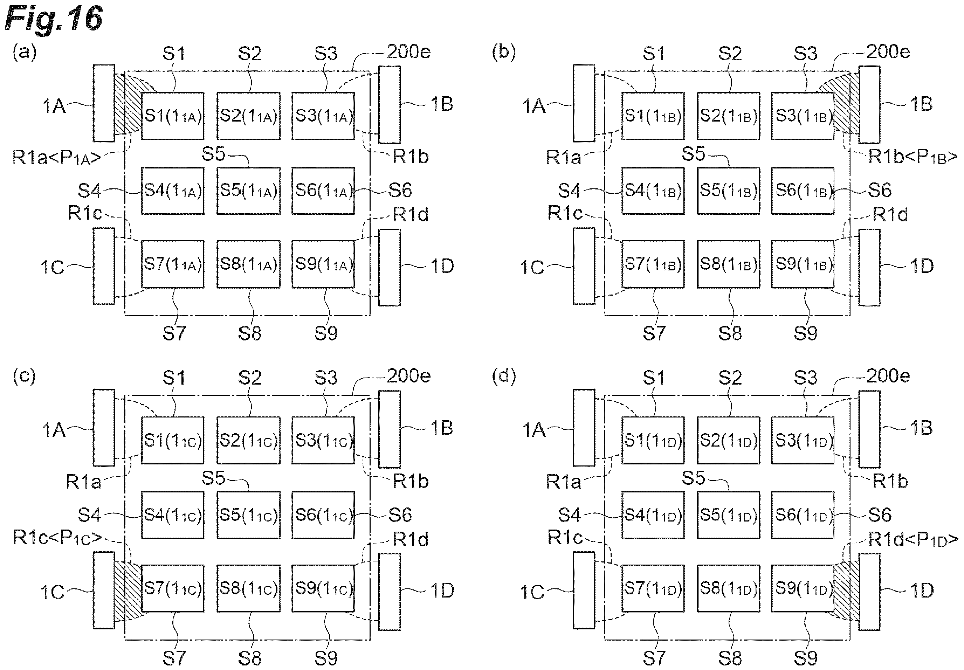

First, as shown in FIG. 16(a), an evaluation value $P_{1A}$ obtained only by the soil evaluation sensor 1A is processed. For the segments S1 to S9, weighting coefficients $S1(1_{1A})$ to $S9(1_{1A})$ are set as the degree of influence on the evaluation value $P_{1A}$ obtained only from the soil evaluation sensor 1A. Therefore, for the segments S1 to S9, the evaluation value $P_{1A}$ is multiplied by the weighting coefficients $S1(1_{1A})$ to $S9(1_{1A})$. As a result, display value components ($P_{1A} \times S1(1_{1A})$ to $P_{1A} \times S9(1_{1A})$) (calculation values) are obtained for the segments S1 to S9.

The same calculation is performed for the soil evaluation sensors 1B, 1C, and 1D. As a result, display value components relevant to the soil evaluation sensor 1B ($P_{1B} \times S1(1_{1B})$ to $P_{1B} \times S9(1_{1B})$), display value components relevant to the soil evaluation sensor 1C ($P_{1C} \times S1(1_{1C})$ to $P_{1C} \times S9(1_{1C})$), and display value components ($P_{1D} \times S1(1_{1D})$ to $P_{1D} \times S9(1_{1D})$) relevant to the soil evaluation sensor 1D are obtained.

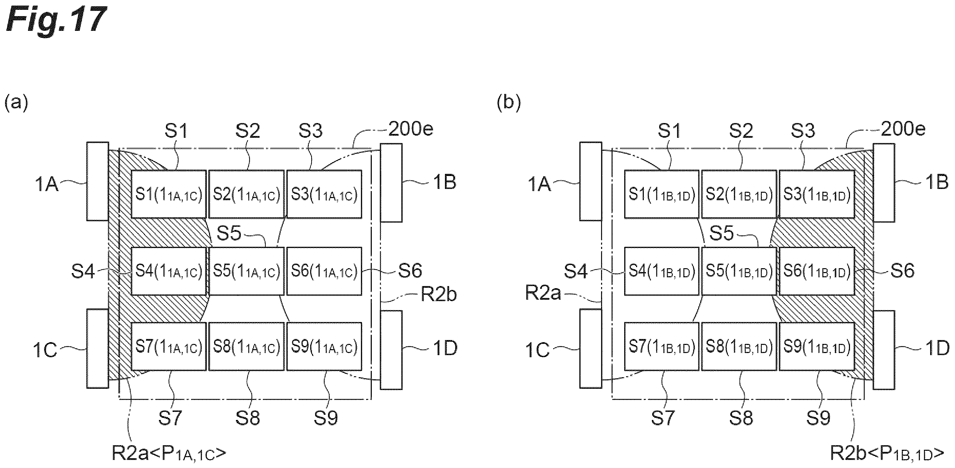

Then, an evaluation value $P_{1A, 1C}$ obtained by the cooperative operation of the soil evaluation sensors 1A and 1C is processed (see FIG. 17(a)). In addition, an evaluation value $P_{1B, 1D}$ obtained by the cooperative operation of the soil evaluation sensors 1B and 1D is processed (see FIG. 17(b)). These calculations are the same as the processing of the evaluation value $P_{1A}$. As a result, display value components ($P_{1A, 1C} \times S1(2_{1A, 1C})$ to $P_{1A, 1C} \times S9(2_{1A, 1C})$) relevant to the soil evaluation sensors 1A and 1C are obtained. In addition, display value components ($P_{1B, 1D} \times S1(2_{1B, 1D})$ to $P_{1B, 1D} \times S9(2_{1B, 1D})$) relevant to the soil evaluation sensors 1B and 1D are obtained.

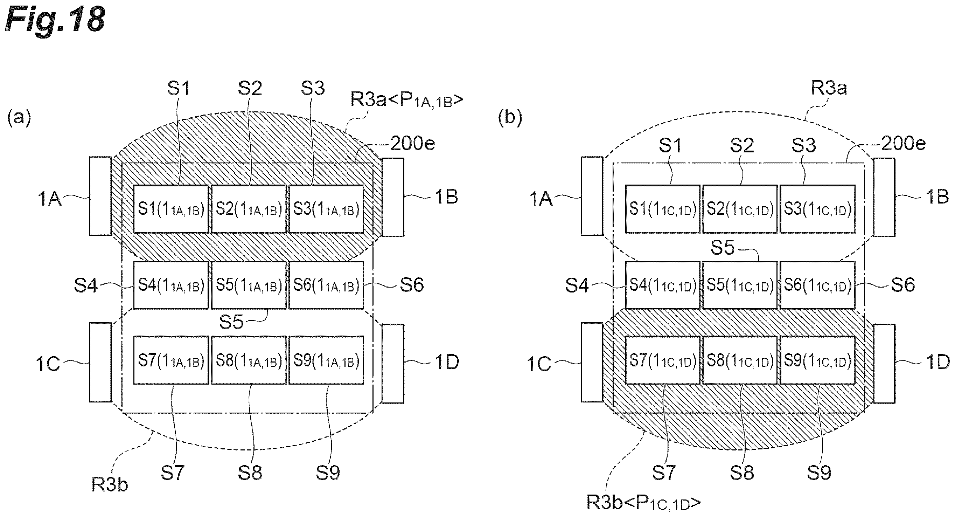

Then, an evaluation value $P_{1A, 1B}$ obtained by the cooperative operation of the soil evaluation sensors 1A and 1B is processed (see FIG. 18(a)). In addition, an evaluation value $P_{1C, 1D}$ obtained by the cooperative operation of the soil evaluation sensors 1C and 1D is processed (see FIG. 18(b)). These calculations are the same as the processing of the evaluation value $P_{1A}$. As a result, display value components ($P_{1A, 1B} \times S1(3_{1A, 1B})$ to $P_{1A, 1B} \times S9(3_{1A, 1B})$) relevant to the soil evaluation sensors 1A and 1B are obtained. In addition, display value components ($P_{1C, 1D} \times S1(3_{1C, 1D})$ to $P_{1C, 1D} \times S9(3_{1C, 1D})$) relevant to the soil evaluation sensors 1C and 1D are obtained.

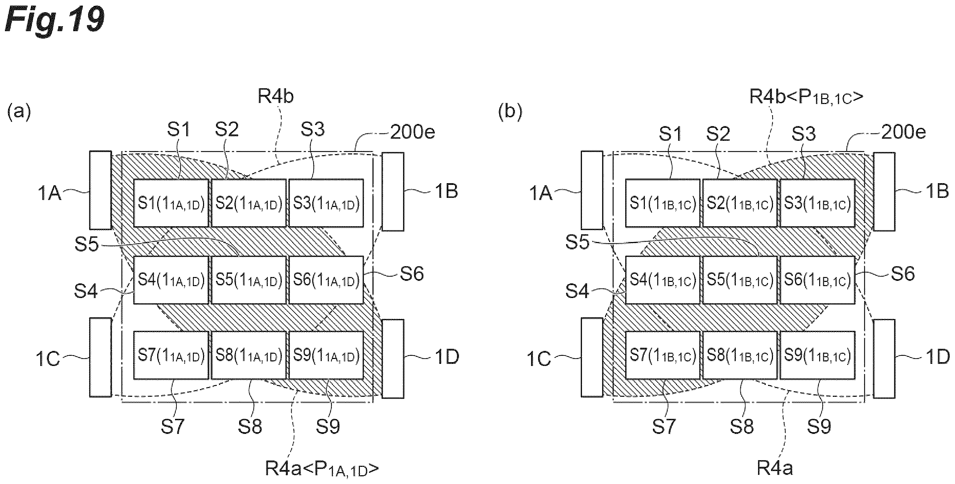

Then, an evaluation value $P_{1A, 1D}$ obtained by the cooperative operation of the soil evaluation sensors 1A and 1D is processed (see FIG. 19(a)). In addition, an evaluation value $P_{1B, 1C}$ obtained by the cooperative operation of the soil evaluation sensors 1B and 1C is processed (see FIG. 19(b)). These calculations are the same as the processing of the evaluation value $P_{1A}$. As a result, display value components ($P_{1A, 1D} \times S1(4_{1A, 1D})$ to $P_{1A, 1D} \times S9(4_{1A, 1D})$) relevant to the soil evaluation sensors 1A and 1D are obtained. In addition, display value components ($P_{1B, 1C} \times S1(4_{1B, 1C})$ to $P_{1B, 1C} \times S9(4_{1B, 1C})$) relevant to the soil evaluation sensors 1B and 1C are obtained.

Then, the sum of the display value components is obtained for each of the segments S1 to S9. The sum of the display value components is a total display value. For example, for the segment S1, ten display value components are obtained. Therefore, by calculating the sum of these, a display value PS1 for the segment S1 can be obtained. The same calculation is performed for the segments S2 to S9.

By the above calculation, a total display value can be obtained for each of the segments S1 to S9.

In addition, in the eighth modification example described above, a display value (total calculation value) is obtained by using a combination of a plurality of sensors shown in FIGS. 17, 18, and 19. The calculation of the display value is not limited to the method described above. For example, at least two or more combinations may be selected from the combinations of the six sensors shown in FIGS. 17, 18, and 19, and the display value may be calculated using the evaluation value based on the selected combination.

In addition, some of the weighting coefficients described above may be determined based on the lines of electric force shown between a sensor that provides a current and a sensor that receives a current. For example, FIG. 18(a) is referred to. The current is provided by the soil sensor 1A and received by the soil sensor 1B. In this case, for example, a linear line of electric force connecting the soil sensors 1A and 1B to each other at the shortest distance can be set between the soil sensors 1A and 1B. The shorter the length of the line of electric force, the smaller the impedance. That is, the degree of influence is large. Therefore, the weighting coefficients $S1(1_{1A, 1B})$, $S2(1_{1A, 1B})$, and $S3(1_{1A, 1B})$ in the segments S1, S2, and S3 crossing the linear line of electric force are set as relatively large values. On the other hand, between the soil sensors 1A and 1B, for example, a curved line of electric force indicated by the outer edge of the measurement range R3a can be set. The length of this line of electric force is longer than the length of the linear line of force. Therefore, the weighting coefficients $S4(1_{1A, 1B})$, $S5(1_{1A, 1B})$, and $S6(1_{1A, 1B})$ in the segments S4, S5, and S6 crossing the linear line of electric force are set as relatively smaller values than the weighting coefficients $S1(1_{1A, 1B})$, $S2(1_{1A, 1B})$, and $S3(1_{1A, 1B})$.

In addition, the setting of the weighting coefficient is not limited to the above-described idea.

Reference Signs List

1, 1A, 1B, 1C, 1D, 1E, 1Ea, 1Eb, 1F, 1G, 1H: soil evaluation sensor, 2, 3: electrode end, 4: voltage generation unit, 6: current measurement unit, 7, 7A:

processing unit, 8: voltage control unit, 9: evaluation value calculation unit, 10, 10A, 10B: soil evaluation system, 11: first calculation unit, 12: second calculation unit, 13: third calculation unit, 14, 14A: control unit, 15, 15A: sensor control unit, 16: display processing unit, 21: switch circuit, 22: switch control unit, 31, 32, 41, 42: extension electrode, 100a: soil, 101: capacitance, 102: electrical resistance.

**Claims**

1. A soil evaluation system for evaluating characteristics of soil in a target region, comprising:

   a first sensor unit that includes a pair of electrode ends disposed in the soil and is adapted to provide an AC input electrical signal to the soil; a second sensor unit that includes a pair of electrode ends disposed in the soil, is adapted to acquire an output electrical signal due to the input electrical signal from the soil, and is spaced apart from the first sensor unit; and a control unit that is adapted to provide a control signal for controlling operations of the first sensor unit and the second sensor unit to the first sensor unit and the second sensor unit, adapted to acquire first information indicating a relationship between a frequency of the input electrical signal and an absolute value of impedance of the soil by using the input electrical signal and the output electrical signal, is adapted to calculate second information indicating a relationship between the frequency of the input electrical signal and a phase of the impedance of the soil by using the first information, and adapted to obtain an evaluation value of the soil by using the first information and the second information, wherein the control unit is adapted to obtain, as the second information, a change in the absolute value of the impedance with respect to a change in the frequency between two points of a first frequency and a second frequency according to a function that is the first information, as an operation for calculating the second information.

2. The soil evaluation system according to claim 1, further comprising:

   a third sensor unit that includes a pair of electrode ends disposed in the soil, is adapted to provide the input electrical signal and acquire the output electrical signal, and is spaced apart from the first sensor unit and the second sensor unit, wherein the first sensor unit is adapted to further acquire the output electrical signal, the second sensor unit is adapted to further provide the input electrical signal, and the first sensor unit, the second sensor unit, and the third sensor unit are disposed so as to surround a target region defined as a two-dimensional surface.

3. The soil evaluation system according to claim 2,

   wherein the control unit performs a first operation of providing the input electrical signal from the first sensor and acquiring the output electrical signal from the second sensor, after the first operation, the control unit performs a second operation of providing the input electrical signal from the first sensor and acquiring the output electrical signal from the third sensor unit, and after the second operation, the control unit performs a third operation of providing the input electrical signal from the second sensor and acquiring the output electrical signal from the third sensor unit.

4. The soil evaluation system according to claim 3,

   wherein the target region includes a plurality of partial regions, a first weighting coefficient, a second weighting coefficient, and a third weighting coefficient are set for the plurality of partial regions, and the control unit performs an operation of obtaining a first calculation value by using the first weighting coefficient and the evaluation value obtained as a result of the first operation, an operation of obtaining a second calculation value by using the second weighting coefficient and the evaluation value obtained as a result of the second operation, an operation of obtaining a third calculation value by using the third weighting coefficient and the evaluation value obtained as a result of the third operation, and an operation of obtaining a total calculation value for each of the partial regions by using the first calculation value, the second calculation value, and the third calculation value obtained for each of the partial regions.

5. The soil evaluation system according to claim 1, further comprising:

   a third sensor unit that includes a pair of electrode ends disposed in the soil, is adapted to provide the input electrical signal and acquire the output electrical signal, and is spaced apart from the first sensor unit and the second sensor unit; and a fourth sensor unit that includes a pair of electrode ends disposed in the soil, is adapted to provide the input electrical signal and acquire the output electrical signal, and is spaced apart from the first sensor unit, the second sensor unit, and third sensor unit,

wherein the first sensor unit is adapted to further acquire the output electrical signal,

the second sensor unit is adapted to further provide the input electrical signal, and

the first sensor unit, the second sensor unit, the third sensor unit, and the fourth sensor unit are disposed so as to surround a target region defined as a three-dimensional region.

**Patentansprüche**

1. Bodenbewertungssystem zum Bewerten von Bodeneigenschaften in einem Zielgebiet, umfassend:

    eine erste Sensoreinheit, die ein Paar Elektrodenenden beinhaltet, die in dem Boden angeordnet sind, und die angepasst ist, um ein elektrisches Wechselstrom-Eingangssignal an den Boden bereitzustellen;

    eine zweite Sensoreinheit, die ein Paar Elektrodenenden beinhaltet, die in dem Boden angeordnet sind, die angepasst ist, um ein elektrisches Ausgangssignal aufgrund des elektrischen Eingangssignals aus dem Boden zu erfassen, und die von der ersten Sensoreinheit beabstandet ist; und

    eine Steuereinheit, die angepasst ist,

    um der ersten Sensoreinheit und der zweiten Sensoreinheit ein Steuersignal für Steueroperationen der ersten Sensoreinheit und der zweiten Sensoreinheit bereitzustellen, die angepasst ist,

    um unter Verwendung des elektrischen Eingangssignals und des elektrischen Ausgangssignals erste Informationen zu erfassen, die eine Beziehung zwischen einer Frequenz des elektrischen Eingangssignals und einem Absolutwert der Impedanz des Bodens angeben, die angepasst ist,

    um unter Verwendung der ersten Informationen zweite Informationen zu berechnen, die eine Beziehung zwischen der Frequenz des elektrischen Eingangssignals und einer Phase der Impedanz des Bodens angeben,

    und die angepasst ist,

    um unter Verwendung der ersten Informationen und der zweiten Informationen einen Bewertungswert des Bodens zu erhalten,

    wobei die Steuereinheit angepasst ist, um als die zweiten Informationen eine Änderung des Absolutwerts der Impedanz in Bezug auf eine Änderung der Frequenz zwischen zwei Punkten einer ersten Frequenz und einer zweiten Frequenz gemäß einer Funktion, die die ersten Informationen sind, als eine Operation zum Berechnen der zweiten Informationen zu erhalten.

2. Bodenbewertungssystem nach Anspruch 1, weiter umfassend:

    eine dritte Sensoreinheit, die ein Paar Elektrodenenden beinhaltet, die in dem Boden angeordnet sind, die angepasst ist, um das elektrische Eingangssignal bereitzustellen und das elektrische Ausgangssignal zu erfassen und von der ersten Sensoreinheit und der zweiten Sensoreinheit beabstandet ist,

    wobei die erste Sensoreinheit angepasst ist, um weiter das elektrische Ausgangssignal zu erfassen,

    der zweite Sensor angepasst ist, um weiter das elektrische Eingangssignal bereitzustellen, und

    die erste Sensoreinheit, die zweite Sensoreinheit und die dritte Sensoreinheit so angeordnet sind, dass sie ein Zielgebiet umgeben, das als eine zweidimensionale Oberfläche definiert ist.

3. Bodenbewertungssystem nach Anspruch 2,

    wobei die Steuereinheit eine erste Operation eines Bereitstellens des elektrischen Eingangssignals von dem ersten Sensor und eines Erfassens des elektrischen Ausgangssignals von dem zweiten Sensor ausführt,

    die Steuereinheit nach der ersten Operation eine zweite Operation eines Bereitstellens des elektrischen Eingangssignals von dem ersten Sensor und eines Erfassens des elektrischen Ausgangssignals von der dritten Sensoreinheit ausführt, und

    die Steuereinheit nach der zweiten Operation eine dritte Operation eines Bereitstellens des elektrischen Eingangssignals von dem zweiten Sensor und eines Erfassens des elektrischen Ausgangssignals von der dritten Sensoreinheit ausführt.

4. Bodenbewertungssystem nach Anspruch 3,

    wobei das Zielgebiet eine Vielzahl von Teilgebieten beinhaltet,

    ein erster Gewichtungskoeffizient, ein zweiter Gewichtungskoeffizient und ein dritter Gewichtungskoeffizient für die Vielzahl von Teilgebieten festgelegt sind, und

    die Steuereinheit eine Operation des Erhaltens eines ersten Berechnungswerts unter Verwendung des ersten Gewichtungskoeffizienten und des als Ergebnis der ersten Operation erhaltenen Bewertungswerts, eine Operation des Erhaltens eines zweiten Berechnungswertes unter Verwendung des zweiten Gewichtungskoeffizienten und des als Ergebnis der zweiten Operation erhaltenen Bewertungswerts, eine Operation des Erhaltens eines dritten Berechnungswerts unter Verwendung des dritten Gewichtungskoeffizienten und des als Ergebnis des dritten Vorgangs erhaltenen Bewertungswerts, und eine Operation des Erhaltens eines Gesamtberechnungswerts für jedes der Teilgebiete unter Verwendung des ersten Berechnungswerts, des zweiten Berechnungswerts und des dritten Berechnungswerts, die für jedes der Teilgebiete erhalten werden, ausführt.

**5.** Bodenbewertungssystem nach Anspruch 1, weiter umfassend:

eine dritte Sensoreinheit, die ein Paar Elektrodenenden beinhaltet, die im Boden angeordnet sind, die angepasst ist, um das elektrische Eingangssignal bereitzustellen und das elektrische Ausgangssignal zu erfassen, und von der ersten Sensoreinheit und der zweiten Sensoreinheit beabstandet ist; und eine vierte Sensoreinheit, die ein Paar Elektrodenenden beinhaltet, die im Boden angeordnet sind, die angepasst ist, um das elektrische Eingangssignal bereitzustellen und das elektrische Ausgangssignal zu erfassen, und von der ersten Sensoreinheit, der zweiten Sensoreinheit und der dritten beabstandet ist, wobei die erste Sensoreinheit angepasst ist, um weiter das elektrische Ausgangssignal zu erfassen, die zweite Sensoreinheit angepasst ist, um weiter das elektrische Eingangssignal bereitzustellen, und die erste Sensoreinheit, die zweite Sensoreinheit, die dritte Sensoreinheit und die vierte Sensoreinheit so angeordnet sind, dass sie ein Zielgebiet umgeben, das als eine dreidimensionale Oberfläche definiert ist.

## Revendications

**1.** Système d'évaluation des sols pour évaluer des caractéristiques de sol dans une région cible, comprenant :

une première unité capteur qui comporte une paire d'extrémités d'électrode disposées dans le sol et qui est adaptée pour fournir un signal électrique d'entrée en courant alternatif au sol ; une deuxième unité capteur qui comporte une paire d'extrémités d'électrode disposées dans le sol, est adaptée pour acquérir un signal électrique de sortie dû au signal électrique d'entrée à partir du sol, et est espacée de la première unité capteur ; et une unité de commande qui est adaptée pour fournir un signal de commande pour commander des opérations de la première unité capteur et de la deuxième unité capteur à la première unité capteur et à la deuxième unité capteur, adaptée pour acquérir une première information indiquant une relation entre une fréquence du signal électrique d'entrée et une valeur absolue d'impédance du sol en utilisant le signal électrique d'entrée et le signal électrique de sortie, est adaptée pour calculer une deuxième information indiquant une relation entre la fréquence du signal électrique d'entrée et une phase de l'impédance du sol en utilisant la première information, et adaptée pour obtenir une valeur d'évaluation du sol en utilisant la première information et la deuxième information, dans lequel l'unité de commande est adaptée pour obtenir, en tant que deuxième information,

un changement de la valeur absolue de l'impédance par rapport à un changement de la fréquence entre deux points d'une première fréquence et d'une deuxième fréquence selon une fonction qui est la première information, en tant qu'une opération de calcul de la deuxième information.

**2.** Système d'évaluation des sols selon la revendication 1, comprenant en outre :

une troisième unité capteur qui comporte une paire d'extrémités d'électrode disposées dans le sol, est adaptée pour fournir le signal électrique d'entrée et acquérir le signal électrique de sortie, et est espacée de la première unité capteur et de la deuxième unité capteur, dans lequel la première unité capteur est adaptée pour acquérir en outre le signal électrique de sortie, la deuxième unité capteur est adaptée pour fournir en outre le signal électrique d'entrée, et la première unité capteur, la deuxième unité capteur, et la troisième unité capteur sont disposées de manière à entourer une région cible définie comme une surface bidimensionnelle.

**3.** Système d'évaluation des sols selon la revendication 2,

dans lequel l'unité de commande réalise une première opération de fourniture du signal électrique d'entrée à partir du premier capteur et d'acquisition du signal électrique de sortie à partir du deuxième capteur,

après la première opération, l'unité de commande réalise une deuxième opération de fourniture du signal électrique d'entrée à partir du premier capteur et d'acquisition du signal électrique de sortie à partir de la troisième unité capteur, et après la deuxième opération, l'unité de commande réalise une troisième opération de fourniture du signal électrique d'entrée à partir du deuxième capteur et d'acquisition du signal électrique de sortie à partir de la troisième unité capteur.

4. Système d'évaluation des sols selon la revendication 3,

dans lequel la région cible comporte une pluralité de régions partielles,
un premier coefficient de pondération, un deuxième coefficient de pondération, et un troisième coefficient de pondération sont établis pour la pluralité des régions partielles, et
l'unité de commande réalise une opération d'obtention d'une première valeur de calcul en utilisant le premier coefficient de pondération et la valeur d'évaluation obtenue à la suite de la première opération, une opération d'obtention d'une deuxième valeur de calcul en utilisant le deuxième coefficient de pondération et la valeur d'évaluation obtenue à la suite de la deuxième opération, une opération d'obtention d'une troisième valeur de calcul en utilisant le troisième coefficient de pondération et la valeur d'évaluation obtenue à la suite de la troisième opération, et une opération d'obtention d'une valeur de calcul totale pour chacune des régions partielles en utilisant la première valeur de calcul, la deuxième valeur de calcul, et la troisième valeur de calcul obtenues pour chacune des régions partielles.

5. Système d'évaluation des sols selon la revendication 1, comprenant en outre :

une troisième unité capteur qui comporte une paire d'extrémités d'électrode disposées dans le sol, est adaptée pour fournir le signal électrique d'entrée et acquérir le signal électrique de sortie, et est espacée de la première unité capteur et de la deuxième unité capteur ; et
une quatrième unité capteur qui comporte une paire d'extrémités d'électrode disposées dans le sol, est adaptée pour fournir le signal électrique d'entrée et acquérir le signal électrique de sortie, et est espacée de la première unité capteur, de la deuxième unité capteur, et de la troisième unité capteur,
dans lequel la première unité capteur est adaptée pour acquérir en outre le signal électrique de sortie,
la deuxième unité capteur est adaptée pour fournir en outre le signal électrique d'entrée, et
la première unité capteur, la deuxième unité capteur, la troisième unité capteur, et la quatrième unité capteur sont disposées de manière à entourer une région cible définie comme une surface tridimensionnelle.

*Fig.1*

*Fig.2*

(a)

(b)

(c)

*Fig.3*

EP 3 872 483 B1

*Fig.4*

(a)

1A  100b  1B
R1a  R1b
1C  1D
R1c  R1d

(b)

1A  100b  1B
R2a  R2b
1C  1D

(c)

R3a  100b
1A  1B
1C  1D
R3b

(d)

R4a  R4b  100b
1A  1B
1C  1D

(e)

R4a  R4b  R3a  100b
1A  1B
R1a  R1b
R1c  R1d
R2a  R2b
1C  1D
R3b

EP 3 872 483 B1

**Fig.5**

Fig.6

Fig.7

EP 3 872 483 B1

Fig.8

Fig.9

# *Fig.10*

## Fig.11

(a)

(b)

**Fig.12**

EP 3 872 483 B1

*Fig.13*

(a)

110

1A

1B

Y

X

Z

1C

1D

200a

(b)

110

Y

X

Z

1A

1B

1C

1D

200b

## Fig.14

*Fig.15*

Fig.16

*Fig.17*

Fig.18

EP 3 872 483 B1

Fig.19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013200193 A